(19) Europäisches Patentamt
European Patent Office
Office européen des brevets

(11) Veröffentlichungsnummer: **0 193 875**
**A2**

# (12) EUROPÄISCHE PATENTANMELDUNG

(21) Anmeldenummer: **86102556.7**

(22) Anmeldetag: **27.02.86**

(51) Int. Cl.[4]: **C07D 295/10**, C07C 97/10, C07D 295/08, C07D 295/12, C07C 101/12, A01N 35/02, A01N 33/04, A01N 39/00, A01N 35/10, A01N 43/10, A01N 43/12

(30) Priorität: **08.03.85 DE 3508398**

(43) Veröffentlichungstag der Anmeldung: **10.09.86 Patentblatt 86/37**

(84) Benannte Vertragsstaaten: **AT CH DE FR GB IT LI**

(71) Anmelder: **HOECHST AKTIENGESELLSCHAFT**
**Postfach 80 03 20**
**D-6230 Frankfurt am Main 80(DE)**

(72) Erfinder: **Tammer, Thomas, Dr.**
**Höchster Strasse 22**
**D-6238 Hofheim am Taunus(DE)**
Erfinder: **Sachse, Burkhard, Dr.**
**An der Ziegelei 30**
**D-6233 Kelkheim (Taunus)(DE)**
Erfinder: **Hartz, Peter, Dr.**
**An der Ziegelei 28**
**D-6233 Kelkheim (Taunus)(DE)**

## (54) Fungizide Mittel auf der Basis von 3-(Hetero)-Arylpropylaminen sowie neue (Hetero)-Arylpropylamine und Verfahren zu ihrer Herstellung.

(57) Gegenstand der Erfindung sind fungizide Mittel, gekennzeichnet durch einen Gehalt einer Verbindung der allgemeinen Formel I,

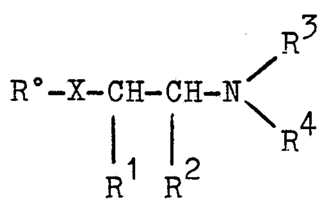

(I)

worin

R° = subst. Phenyl oder andere (substituierte) Aromaten- und Heteroaromatenreste,

X = $>C=O$, $>CH(OR^5)$, $>C=N-OR^5$, 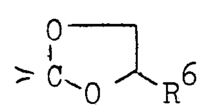

$>C=N-NH-CS-NH_2$ oder $>C=N-NH-CO-NH_2$,

EP 0 193 875 A2

$R^1$ =H, Cl, Br, (subst.) Alkyl; $R^2$ = H, Cl, Br, Alkyl, -(subst.) Phenyl, Thienyl oder Furanyl,

$R^3$ und $R^4$ = H, (subst.) Alkyl, 2,2,6,6,-Tetramethylpiperidin-4-yl, Alkenyl, Alkinyl oder Cycloalkyl, oder $R^3$ und $R^4$ zusammen mit dem Stickstoffatom, an das sie geknüpft sind, eine (substituierten) (durch eine ($C_1$-$C_2$)-Alkylenkette überbrückten) 3-bis 9-gliedrigen gesättigten Heterocyclenring, der als Ringglied auch ein O-oder S-Atom oder die Gruppe $NR^7$, -CH=CH-, C=O oder -CH=N enthalten kann, oder (subst.) 1,2,3,4-Tetrahydroisochinolin, oder (subst.) 8-Aza-1,4-dioxa-spiro-(4,5)decan bedeuten.

Zahlreiche Verbindungen aus dieser Gruppe sind neu und werden ebenfalls von der Erfindung erfaßt. Die Verbindungen der Formel I besitzen eine breite fungizide Wirksamkeit gegen phytopathogene Pilze.

# Fungizide Mittel auf der Basis von 3-(Hetero)-Arylpropylaminen sowie neue (Hetero)-Arylpropylamine und Verfahren zu ihrer Herstellung

Die vorliegende Erfindung betrifft die Verwendung von 3-(Hetero)Aryl-propylaminen zur Bekämpfung von Schadpilzen. 3-Arylpropylamine, die fungizid wirksam sind, sind aus den Deutschen Offenlegungsschriften DE-OS 30 19 497 und DE-OS 30 34 383 bekannt. Die Wirkung dieser Verbindungen gegenüber phytopathogenen Pilzen ist jedoch im Hinblick auf die Breite des Wirkungsspektrums bei gleichzeitig hohem Wirkungsgrad unzureichend.

Es wurden nun 3-(Hetero)Aryl-propylamine mit vorteilhaften fungiziden Eigenschaften gefunden. Einige dieser Verbindungen sind aus US-PS 3 462 036 und DE-OS 28 42 759 bekannt. Für diese Verbindungen werden jedoch lediglich pharmazeutische Wirkungen angegeben.

Gegenstand der vorliegenden Erfindung sind daher fungizide Mittel, gekennzeichnet durch einen wirksamen Gehalt einer Verbindung der allgemeinen Formel I,

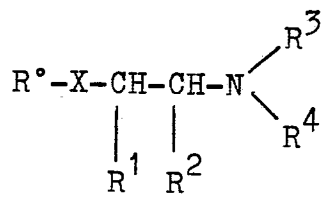

(I)

worin

R° = Phenyl, das ein-oder mehrfach substituiert ist durch Halogen, Nitro, Hydroxy, Acetoxy, ($C_1$-$C_{12}$)-Alkyl, das gegebenenfalls ein-oder mehrfach durch Halogen, Hydroxy, ($C_1$-$C_4$)-Alkoxy, Phenoxy oder Phenyl substituiert ist, die beide ein-oder·mehrfach durch ($C_1$-$C_4$)-Alkyl, Halogen, Nitro oder $CF_3$ substituiert sein können, oder

durch ($C_3$-$C_7$)-Cycloalkyl, das ein-oder mehrfach durch Halogen, ($C_1$-$C_4$)-Alkyl, ($C_1$-$C_4$)-Alkoxy, Phenyl oder [($C_1$-$C_4$) alkoxy]carbonyl substituiert sein kann, oder

durch ($C_1$-$C_8$)-Alkoxy, ($C_1$-$C_2$)-Alkoxyethoxy, ($C_1$-$C_8$)-Alkylthio), ($C_1$-$C_8$)-Halogenalkoxy, ($C_1$-$C_8$)-Halogenalkylthio, ($C_2$-$C_4$)-Alkenyl, ($C_2$-$C_6$)-Alkenoxy, oder durch Phenyl, das ein-oder mehrfach durch Halogen, $CF_3$ oder $NO_2$ substituiert sein kann, oder

durch Phenoxy, Benzyloxy, Naphthyloxy, Phenylthio oder Phenylsulfonyl, die alle ein-oder mehrfach durch CN, $NH_2$, $NO_2$, COO($C_1$-$C_4$)alkyl, Halogen, ($C_1$-$C_4$)-Halogenalkyl, ($C_1$-$C_4$)-Alkyl, ($C_1$-$C_4$)-Alkoxy, ($C_1$-$C_4$)-Alkylthio oder ($C_1$-$C_4$)-Halogenalkoxy, substituiert sein können,

oder durch Pyridyloxy, Chinolyloxy oder Chinoxalyloxy, die alle ein-oder mehrfach durch CN, Halogen, ($C_1$-$C_4$)-Alkyl, ($C_1$-$C_4$)-Alkoxy, ($C_1$-$C_4$)-Halogenalkyl, ($C_1$-$C_4$)-Halogenalkoxy, Hydroxy oder Nitro substituiert sein können,

Phenyl, das an benachbarten Positionen durch ($C_1$-$C_2$)-Alkylendioxy substituiert ist,

Naphthyl, Indanyl oder 1,2,3,4-Tetrahydronaphthyl, die alle ein-oder mehrfach durch Halogen, Hydroxy, ($C_1$-$C_4$)-Alkyl, -($C_1$-$C_4$)-Alkoxy, ($C_1$-$C_4$)-Halogenalkyl oder Phenoxy, das ein-oder mehrfach durch Halogen, ($C_1$-$C_4$)-Alkyl, $NO_2$, ($C_1$-$C_4$)-Alkoxy, ($C_1$-$C_4$)-Halogenalkyl oder ($C_1$-$C_4$)-Halogenalkoxy substituiert sein kan, substituiert sein können,

Fluorenyl oder 9,10-Dihydroanthracenyl, die beide ein-oder mehrfach durch Halogen, Hydroxy, $NO_2$, ($C_1$-$C_4$)-Alkoxy, -($C_1$-$C_4$)-Alkyl, ($C_1$-$C_4$)-Halogenalkyl oder Phenoxy, das ein-oder mehrfach durch Halogen, ($C_1$-$C_4$)-Alkyl, CN, $NO_2$ oder $CF_3$ substituiert sein kann, substituiert sein können,

Dibenzofuranyl oder Dibenzothienyl, die beide ein-bis dreifach durch Halogen, Hydroxy, $NO_2$, ($C_1$-$C_4$)-Alkyl oder ($C_1$-$C_4$)-Alkoxy substituiert sein können,

Furanyl oder Thienyl, die beide ein-oder mehrfach durch Halogen, Nitro, ($C_1$-$C_4$)-Alkyl, ($C_1$-$C_4$)-Alkoxy, ($C_1$-$C_4$)-Hydroxyalkyl, ($C_1$-$C_4$)-Alkoxy-($C_1$-$C_2$)-alkyl, Phenyl, Benzyl, Phenoxy oder Phenylthio, wobei diese Arylreste ein-oder mehrfach durch Halogen, $CF_3$, $NO_2$, CN, ($C_1$-$C_4$)-Alkyl oder ($C_1$-$C_4$)-Alkylcarbonyl substituiert sein können, substituiert sein können,

Pyridyl, das ein-oder mehrfach durch Halogen, $NO_2$, CN, -($C_1$-$C_8$)-Alkyl, ($C_1$-$C_4$)-Halogenalkyl, ($C_1$-$C_4$)-Alkoxy, ($C_1$-$C_4$)-Halogenalkoxy, Phenoxy, das ein-oder mehrfach durch $CF_3$, $NO_2$, Halogen, ($C_1$-$C_4$)alkoxycarbonyl, ($C_1$-$C_4$)-Alkyl oder CN substituiert sein kann, Phenyl oder Phenyl, das ein-oder mehrfach durch Halogen substituiert ist, substituiert sein kann,

Benzofuranyl, Benzothienyl oder Benzofuranyl, Benzothienyl, die beide ein-bis drei fach durch Halogen, Nitro, ($C_1$-$C_4$)Alkyl, ($C_1$-$C_4$)-Halogenalkyl oder ($C_1$-$C_4$)-Alkoxy substituiert sind,

$$X = \; >C=O, \; >CH(OR^5), \; >C=N-OR^5,$$ 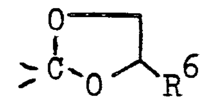

$$>C=N-NH-CS-NH_2 \quad \text{oder} \quad >C=N-NH-CO-NH_2,$$

$R^1$ = H, Cl, Br, ($C_1$-$C_4$)-Alkyl oder ($C_1$-$C_4$)-Alkyl, das ein-oder mehrfach durch Halogen, ($C_1$-$C_4$)-Alkoxy oder Phenyl, das ein-oder mehrfach durch Halogen substituiert sein kann, substituiert ist,

$R^2$ = H, Cl, Br, ($C_1$-$C_4$)-Alkyl, Phenyl, Phenyl, das ein-oder mehrfach durch Halogen, Nitro, Hydroxy, ($C_1$-$C_4$)-Halogenalkyl, ($C_1$-$C_4$)-Alkoxy oder Phenoxy substituiert ist, Thienyl oder Furanyl,

$R^3$ und $R^4$ unabhängig voneinander

= H, ($C_1$-$C_4$)-Alkyl, ($C_1$-$C_4$)-Alkyl, das ein-oder mehrfach durch halogen, CN, Hydroxy, ($C_1$-$C_4$)-Alkoxy, Phenoxy oder Phenyl, wobei diese Arylreste ein-oder mehrfach durch Halogen, $CF_3$, $NO_2$, oder ($C_1$-$C_4$)-Alkyl substituiert sein können, ($C_1$-$C_4$-Alkoxy)-carbonyl, Carboxy, ($C_1$-$C_4$)-Alkylthio, ($C_1$-$C_4$)-Alkylsulfinyl, ($C_1$-$C_4$)-Alkylsulfonyl, ($C_3$-$C_7$)-Cycloalkyl, ($C_1$-$C_4$)-Halogenalkoxy, ($C_1$-$C_4$)-Alkylamino oder Bis-($C_1$-$C_4$-alkyl)amino substituiert ist,

2,2,6,6-Tetramethylpiperidin-4-yl, ($C_2$-$C_4$)-Alkenyl, ($C_2$-$C_4$)-Alkinyl oder ($C_3$-$C_7$)-Cycloalkyl, oder

$R^3$ und $R^4$ zusammen mit dem Stickstoffatom, an das sie geknüpft sind,

einen gegebenenfalls ein-oder mehrfach durch ($C_1$-$C_4$)-Alkyl, Hydroxy, ($C_1$-$C_2$)-Hydroxyalkyl, Carboxy, ($C_1$-$C_4$-alkoxy)-carbonyl, Phenyl oder Benzyl, die beide ein-oder mehrfach durch Halogen, ($C_1$-$C_4$)-Alkyl, ($C_1$-$C_4$)-Alkoxy, $NO_2$, CN, $CF_3$ oder Hydroxy substituiert sein können, oder durch ($C_1$-$C_2$)-Alkoxy-($C_1$-$C_2$)-alkyl substituierten und/oder durch eine ($C_1$-$C_2$)-Alkylenkette einfach überbrückten 3-bis 9-gliedrigen gesättigten Heterocyclenring, der als Ringglied auch ein O- oder S-Atom oder die Gruppe $NR^7$, -CH=CH-, >C=O oder -CH=N-enthalten kann,

oder 1,2,3,4-Tetrahydroisochinolin, das ein-oder mehrfach durch ($C_1$-$C_4$)-Alkyl, Hydroxy, ($C_1$-$C_2$)-Hydroxyalkyl, ($C_1$-$C_4$)-Alkoxy, ($C_1$-$C_4$)-Halogenalkyl oder Halogen substituiert sein kann, oder 8-Aza-1,4-dioxa-spiro(4,5)decan, das ein-oder mehrfach durch ($C_1$-$C_4$)-Alkyl, ($C_1$-$C_4$-Alkoxy)carbonyl, Carboxy oder ($C_1$-$C_2$)-Halogenalkyl substituiert sein kann, bedeuten,

$R^5$ = H, ($C_1$-$C_{12}$)-Alkyl, ($C_2$-$C_6$)-Alkenyl, ($C_2$-$C_4$)-Alkinyl, Benzyl, das im Aromatenteil ein-oder mehrfach durch Halogen, ($C_1$-$C_4$)-Alkyl, ($C_1$-$C_2$)-Halogenalkyl, ($C_1$-$C_4$)-Alkoxy, CN, $NO_2$ oder Hydroxy substituiert sein kann, Pyridyl oder Pyridyl, das ein-oder mehrfach durch Halogen, $CF_3$, $CCl_3$ oder -($C_1$-$C_4$)-Alkyl substituiert ist,

$R^6$ = H, ($C_1$-$C_8$)-Alkyl oder ($C_1$-$C_2$)-Halogenalkyl, und

$R^7$ = H, ($C_1$-$C_{12}$)-Alkyl, ($C_1$-$C_{12}$)-Alkyl, das gegebenenfalls ein-oder mehrfach durch Hydroxy oder ($C_1$-$C_2$)-Alkoxy substituiert ist, Phenyl, Benzyl oder Pyridyl, die alle ein-oder mehrfach durch Halogen, $NO_2$, OH, CN, ($C_1$-$C_4$)-Alkyl, ($C_1$-$C_2$)-Halogenalkyl oder ($C_1$-$C_4$)-Alkoxy substituiert sein können, oder den Rest -$CHR^2$-$CHR^1$-X-$R^\circ$, wobei die Substituenten $R^\circ$, $R^1$, $R^2$ und X in der jeweiligen konkreten Verbindung der Formel I die identisch-selben Bedeutungen wie die obengenannten Reste $R^\circ$, $R^1$, $R^2$ und X besitzen sollen, bedeutet, sowie ihre Salze,

mit der Maßgabe, daß im Falle

a) $NR^3R^4$ = 2,6-Di-($C_1$-$C_4$-alkyl)morpholino, X = >C=O oder $CH(OR^5)$ und $R^\circ$ ein substituierter Phenylring bedeutet, der einen Rest der Gruppe Halogen, ($C_1$-$C_6$)-Alkyl, ($C_5$-$C_6$)-Cycloalkyl, ($C_1$-$C_8$)-Alkoxy, ($C_2$-$C_6$)-Alkenoxy, Phenoxy oder Phenyl, wobei diese Arylreste ein-oder mehrfach durch Halogen substituiert sein können, enthält, oder im Falle

b) X = >C=O oder >C=N-$OR^5$, $R^1$ = H oder -($C_1$-$C_4$)-Alkyl, $R^2$ = H und $R^\circ$ ein substituierter Phenylring bedeutet, der einen Rest der Gruppe Halogen, ($C_1$-$C_4$)-Alkyl, ($C_1$-$C_2$)-Halogenalkyl, ($C_3$-$C_7$)-Cycloalkyl, ($C_1$-$C_2$)-Alkoxy, ($C_1$-$C_2$)-Halogenalkoxy, ($C_1$-$C_2$)-Alkylthio oder ($C_1$-$C_2$)-Halogenalkylthio enthält, der Phenylring von $R^\circ$ des weiteren durch Hydroxy oder Nitro substituiert sein muß, und im Falle

c) $NR^3R^4$ = 2,6-Di-($C_1$-$C_4$-alkyl)morpholino und X = >C=O oder >$CH(OR^5)$ bedeutet, $R^\circ$ keinen unsubstituierten Naphthyl-, Indanyl-oder 1,2,3,4-Tetrahydronaphthyl-Rest bedeuten darf.

Als Salze der Verbindungen der Formel I kommen hier wie im folgenden Salze mit anorganischen Säuren wie HCl, HBr, oder organischen Säuren wie p-Toluolsulfonsäure, Naphthalinsulfonsäuren, insbesondere Salte mit HCl oder HBr in Frage.

Bevorzugt sind fungizide Mittel, die eine Verbindung der Formel I enthalten, wobei

$R^\circ$ = Phenyl, das ein-oder mehrfach substituiert ist durch Halogen, Nitro, Hydroxy, ($C_1$-$C_{12}$)-Alkyl, das gegebenenfalls ein-oder mehrfach halogeniert ist, oder Benzyl, das ein-oder mehrfach durch ($C_1$-$C_4$)-Alkyl, Halogen, Nitro oder $CF_3$ substituiert sein kann, oder durch ($C_3$-$C_7$)-Cycloalkyl, das ein-oder mehrfach durch Halogen, ($C_1$-$C_4$)-Alkyl, oder Phenyl substituiert sein kann, oder

durch ($C_1$-$C_8$)-Alkoxy, ($C_1$-$C_8$)-Halogenalkoxy, oder durch Phenyl, das ein-oder mehrfach durch Halogen, $CF_3$ oder $NO_2$ substituiert sein kann, oder durch Phenoxy, Benzyloxy, Naphthyloxy, Phenylthio oder Phenylsulfonyl, die alle ein-oder mehrfach durch $NO_2$, Halogen, ($C_1$-$C_4$)-Halogenalkyl, ($C_1$-$C_4$)-Alkyl, ($C_1$-$C_4$)-Alkoxy, oder ($C_1$-$C_4$)-Halogenalkoxy, substituiert sein können,

oder durch Pyridyloxy, Chinolyloxy oder Chinoxalyl oxy, die alle ein-oder mehrfach durch Halogen, ($C_1$-$C_4$)-Alkyl, ($C_1$-$C_4$)-Alkoxy, ($C_1$-$C_4$)-Halogenalkyl, ($C_1$-$C_4$)-Halogenalkoxy,

Hydroxy oder Nitro substituiert sein können.

Naphthyl, Indanyl oder 1,2,3,4-Tetrahydronaphthyl, die alle ein-oder zweifach durch Halogen, Hydroxy, $(C_1-C_4)$-Alkyl, -$(C_1-C_4)$-Alkoxy oder $(C_1-C_4)$-Halogenalkyl substituiert sein können,

Fluorenyl, das ein-oder zweifach durch Halogen, $NO_2$, $(C_1-C_4)$-Alkyl oder $(C_1-C_4)$-Halogenalkyl substituiert sein kann,

Dibenzofuranyl oder Dibenzothienyl, die beide ein-bis dreifach durch Halogen, $NO_2$ oder $(C_1-C_4)$-Alkyl substituiert sein können,

Furanyl oder Thienyl, die beide ein-oder zweifach durch Halogen, Nitro, $(C_1-C_4)$-Alkyl, $(C_1-C_4)$-Alkoxy, substituiert sein können,

Pyridyl, das ein-oder zweifach durch Halogen, $NO_2$, $(C_1-C_4)$-Alkyl, $(C_1-C_4)$-Halogenalkyl, Phenoxy, das ein-oder mehrfach durch $CF_3$, $NO_2$ oder Halogen substituiert sein kann, durch Phenyl oder Phenyl, das ein-oder zweifach durch Halogen substituiert ist, substituiert sein kann,

$R^1$, $R^2$ = H, X = $>CO$,

$R^3$ und $R^4$ zusammen mit dem Stickstoffatom, an das sie geknüpft sind, einen gegebenenfalls ein-bis dreifach durch -$(C_1-C_4)$-Alkyl, $(C_1-C_2)$-Hydroxyalkyl, Hydroxy oder $(C_1-C_2)$-Alkoxy-$(C_1-C_2)$-alkyl substituierten 7-bis 9-gliedrigen Heterocyclenring, der zusätzlich die Gruppe $NR^7$ oder -(CH=N)-als Ringglied enthalten kann, und

$R^7$ = H, $(C_1-C_4)$-Alkyl, Phenyl oder Benzyl, die beide ein-bis dreifach durch Halogen, $(C_1-C_4)$-Alkyl oder $(C_1-C_4)$-Alkoxy substituiert sein können, bedeuten,

sowie ihre Salze.

Gegenstand der vorliegenden Erfindung sind auch die neuen 3-(Hetero)-Aryl-propylamine der oben angegebenen Formel I und deren Salze, worin

$R^0$ = Phenyl, das einfach durch $(C_3-C_7)$-Cycloalkyl, Phenyl, Phenoxy, Naphthoxy, Benzyl, Benzyloxy, Phenylthio, Phenylsulfonyl, Pyridyloxy, Chinolyloxy, oder Chinoxalyloxy oder an benachbarten Positionen durch $(C_1-C_2)$-Alkylendioxy, wobei diese Reste ein-oder mehrfach durch Halogen, $(C_1-C_4)$-Alkyl, $(C_1-C_2)$-Halogenalkyl, $(C_1-C_4)$-Alkoxy, $(C_1-C_3)$-Halogenalkoxy, OH, CN oder $NO_2$ substituiert sein können, substituiert ist und ferner ein-bis dreifach durch Halogen, $NO_2$, Hydroxy, $(C_1-C_{12})$-Alkyl, $(C_1-C_{12})$-Alkyl, das ein-oder mehrfach durch Halogen, Hydroxy oder $(C_1-C_4)$-Alkoxy substituiert ist, $(C_1-C_8)$-Alkoxy, $(C_1-C_2)$-Alkoxyethoxy, $(C_1-C_8)$-Alkylthio, $(C_1-C_8)$-Halogenalkyl, $(C_1-C_8)$-Halogenalkoxy, $(C_1-C_8)$-Halogenalkylthio, $(C_2-C_4)$-Alkenyl, $(C_2-C_6)$-Alkenoxy, Phenoxy oder Phenoxy, das ein-oder mehrfach durch Halogen, $CF_3$, $NO_2$ oder $(C_1-C_4)$-Alkyl substituiert ist, substituiert sein kann,

Naphthyl, Indanyl oder 1,2,3,4-Tetrahydronaphthyl, wobei diese drei Reste ein-oder mehrfach durch Halogen, Hydroxy, $(C_1-C_4)$-Alkyl, $(C_1-C_4)$-Alkoxy, $(C_1-C_4)$-Halogenalkyl, Phenoxy oder Phenoxy, das ein-oder mehrfach durch Halogen, $(C_1-C_4)$-Alkyl, $NO_2$, $(C_1-C_4)$-Alkoxy, $(C_1-C_4)$-Halogenalkyl oder $(C_1-C_4)$-Halogenalkoxy substituiert ist, substituiert sein können,

Fluorenyl oder 9,10-Dihydroanthracenyl, die beide ein-oder mehrfach durch Halogen, Hydroxy, $NO_2$, $(C_1-C_4)$-Alkoxy, -$(C_1-C_4)$-Alkyl, $(C_1-C_4)$-Halogenalkyl, Phenoxy oder Phenoxy, das ein-oder mehrfach durch Halogen, $(C_1-C_4)$-Alkyl, CN, $NO_2$ oder $CF_3$ substituiert ist, substituiert sein können,

Dibenzofuranyl oder Dibenzothienyl, die beide ein-bis dreifach durch Halogen, Hydroxy, $NO_2$, $(C_1-C_4)$-Alkyl oder $(C_1-C_4)$-Alkoxy substituiert sein können, Furanyl oder Thienyl, die beide ein-oder mehrfach durch Halogen, Nitro, $(C_1-C_4)$-Alkyl, $(C_1-C_4)$-Hydroxyalkyl, $(C_1-C_4)$-Alkoxy-$(C_1-C_2)$-alkyl, Phenyl, Benzyl, Phenoxy, oder Phenylthio, wobei diese Arylreste ein-oder mehrfach durch Halogen, $CF_3$, $NO_2$, CN, $(C_1-C_4)$-Alkyl oder $(C_1-C_4)$-Alkylcarbonyl substituiert sein können, substituiert sein können, Pyridyl oder Pyridyl, das ein-oder mehrfach durch Halogen, $NO_2$, CN, $(C_1-C_8)$-Alkyl, -$(C_1-C_4)$-Halogenalkyl, $(C_1-C_4)$-Alkoxy, $(C_1-C_4)$-Halogenalkoxy, Phenoxy oder Phenoxy, das ein-oder mehrfach durch Halo gen, $NO_2$, $CF_3$, COO$(C_1-C_4)$-alkyl, CN oder $(C_1-C_4)$-Alkyl substituiert ist, durch Phenyl oder Phenyl, das ein-oder mehrfach durch Halogen substituiert ist, substituiert ist, oder

Benzofuranyl, Benzothienyl, die beide ein-bis dreifach durch Halogen, Nitro, $(C_1-C_4)$-Alkyl, $(C_1-C_4)$-Halogenalkyl oder $(C_1-C_4)$-Alkoxy substituiert sein können,

X = $>C=O$, $>CH(OR^5)$, $>C=NOR^5$, 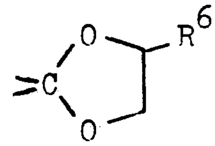 ,

$>C=N-NH-\underset{\underset{S}{\Vert}}{C}-NH_2$ oder $>C=N-NH-\underset{\underset{O}{\Vert}}{C}-NH_2$ ,

$R^1$ = H, Cl, Br, $(C_1-C_4)$-Alkyl oder $(C_1-C_4)$-Alkyl, das ein-oder mehrfach durch Halogen, $(C_1-C_4)$-Alkoxy, Phenyl oder Phenyl, das ein-oder mehrfach durch Halogen substituiert ist, substituiert ist,

$R^2$ = H, Cl, Br, $(C_1-C_4)$-Alkyl, Phenyl oder Phenyl, das ein-oder mehrfach durch Halogen, Nitro, Hydroxy, $(C_1-C_4)$-Alkyl, $(C_1-C_4)$-Halogenalkyl, $(C_1-C_4)$-Alkoxy oder Phenoxy substituiert ist, Thienyl oder Furanyl,

$R^3$ und $R^4$ = zusammen mit dem Stickstoffatom, an das sie geknüpft sind, einen gegebenenfalls ein-oder mehrfach durch $(C_1-C_4)$-Alkyl, Hydroxy, $(C_1-C_2)$-Hydroxyalkyl oder $(C_1-C_4)$-Alkoxy-$(C_1-C_2)$-alkyl substituierten und/oder durch eine

($C_1$-$C_2$)-Alkylenkette einfach überbrückten 7-bis 9-gliedrigen gesättigten Heterocyclenring, der als Ringglied auch ein Element der Gruppe O, S, $NR^7$, (CH=CH) oder (CH=N) enthalten kann, oder

8-Aza-1,4-dioxa-spiro(4,5)decan, das ein-oder mehrfach durch ($C_1$-$C_4$)-Alkyl, COO($C_1$-$C_4$)-alkyl, COOH oder ($C_1$-$C_2$)-Halogenalkyl substituiert sein kann,

$R^5$ = H, ($C_1$-$C_{12}$)-Alkyl, ($C_2$-$C_6$)-Alkenyl, ($C_2$-$C_4$)-Alkinyl, Benzyl oder Benzyl, das im Aromatenteil ein-oder mehrfach durch Halogen, ($C_1$-$C_4$)-Alkyl, ($C_1$-$C_2$)-Halogenalkyl, ($C_1$-$C_4$)-Alkoxy, CN, $NO_2$ oder Hydroxy substituiert ist,

Pyridyl oder Pyridyl, das ein oder mehrfach durch Halogen, $CF_3$, $CCl_3$ oder ($C_1$-$C_4$)-Alkyl substituiert ist,

$R^6$ = H, ($C_1$-$C_8$)-Alkyl oder ($C_1$-$C_2$)-Halogenalkyl, und

$R^7$ = H, ($C_1$-$C_{12}$)-Alkyl, ($C_1$-$C_{12}$)-Alkyl, das ein-oder mehrfach durch Hydroxy oder ($C_1$-$C_2$)-Alkoxy substituiert ist, Phenyl, Benzyl oder Pyridyl, wobei diese Arylreste ein-oder mehrfach durch Halogen, $NO_2$, OH, CN, ($C_1$-$C_4$)-Alkyl, ($C_1$-$C_2$)-Halogenalkyl oder ($C_1$-$C_4$)-Alkoxy substituiert sein können,

wobei für diesen Rest die Substituenten R°, $R^1$, $R^2$ und X jeweils identisch mit den anderen Resten R°, $R^1$, $R^2$ und X der Formel I sein sollen.

Bei allen obengenannten Definitionen für R°, $R^1$, $R^2$, $R^3$, $R^4$, $R^5$, $R^6$ und $R^7$ bedeutet Halogen insbesondere Fluor und Chlor; ($C_1$-$C_8$)-Halogenalkoxy oder ($C_1$-$C_8$)-Halogenalkylthio enthält insbesondere 3-6. Fluor-, Chlor-oder Bromatome und ($C_1$-$C_4$)-Halogenalkyl oder ($C_1$-$C_4$)-Halogenalkoxy enthält insbesondere 1-8 Fluor-, Chlor-oder Bromatome, ($C_1$-$C_3$)-Halogenalkoxy enthält insbesondere 3-6 ($C_1$-$C_2$)-Halogenalkyl enthält insbesondere 1-5 Fluor-, Chlor-oder Bromatome.

Bevorzugt von den neuen Verbindungen der Formel I und deren Salzen sind solche, in denen

R° = Phenyl, das einfach durch Phenyl, Phenoxy, Naphthoxy, Benzyl, Benzyloxy, Phenylthio, Phenylsulfonyl, Pyridyloxy, Chinolyloxy oder Chinoxalyloxy, die alle ein-bis dreifach durch Halogen, ($C_1$-$C_4$)-Alkyl, $CF_3$, ($C_1$-$C_4$)-Alkoxy, Hydroxy, CN oder $NO_2$ substituiert sein können, substituiert ist, und ferner ein-bis zweifach durch Halogen, Hydroxy, ($C_1$-$C_4$)-Alkyl, ($C_1$-$C_4$)-Alkoxy oder Nitro substituiert sein kann,

Phenyl, das einfach durch ($C_3$-$C_7$)-Cycloalkyl, das ein-bis vierfach durch Halogen, Phenyl oder ($C_1$-$C_4$)-Alkyl substituiert ist, oder einfach durch ($C_1$-$C_2$)-Alkylendioxy substituiert ist, wobei Phenyl ferner ein-bis zweifach durch OH, ($C_1$-$C_4$)-Alkoxy, ($C_1$-$C_4$)-Alkyl, Phenoxy oder Phenoxy, das ein-oder mehrfach durch Halogen oder $CF_3$ substituiert ist, substituiert sein kann,

Naphthyl, Indanyl oder 1,2,3,4-Tetrahydronaphthyl, die alle ein-bis zweifach durch Halogen, Hydroxy, ($C_1$-$C_4$)-Alkyl oder ($C_1$-$C_4$)-Alkoxy substituiert sein können,

Fluorenyl, das ein-bis zweifach durch Halogen, $NO_2$ oder -($C_1$-$C_4$)-Alkyl substituiert sein kann,

Dibenzofuranyl oder Dibenzothienyl, die beide ein-bis dreifach durch $NO_2$, Halogen oder ($C_1$-$C_4$)-Alkyl substituiert sein können,

Furanyl oder Thienyl, die beide ein-bis zweifach durch Halogen, Nitro oder ($C_1$-$C_4$)-Alkyl substituiert sein können,

Pyridyl oder Pyridyl, das ein-bis zweifach durch Halogen, $CF_3$, ($C_1$-$C_4$)-Alkyl, Phenoxy oder Phenoxy, das ein-oder mehrfach durch Halogen, $NO_2$ oder $CF_3$ substituiert ist, Phenyl oder Phenyl, das ein-bis zweifach durch Halogen substituiert ist, substituiert ist,

X = $>$ CO; $R^1$, $R^2$ = H,

$R^3$ und $R^4$ = zusammen mit dem Stickstoffatom, an das sie geknüpft sind, einen gegebenenfalls ein-bis dreifach durch -($C_1$-$C_4$)-Alkyl, Hydroxy, ($C_1$-$C_2$)-Hydroxyalkyl oder ($C_1$-$C_2$)-Alkoxy-($C_1$-$C_2$)-alkyl substituierten 7-bis 9-gliedrigen Heterocyclen-Ring bedeutet, der zusätzlich die Gruppe -$NR^7$ oder -(CH=N)-als Ringglied enthalten kann, und

$R^7$ = H, ($C_1$-$C_4$)-Alkyl, Phenyl oder Benzyl, die beide ein-bis dreifach durch Halogen, ($C_1$-$C_4$)-Alkyl oder ($C_1$-$C_4$)-Alkoxy substituiert sein können, bedeuten.

Gegenstand der vorliegenden Erfindung sind auch Verfahren zur Herstellung der neuen Verbindungen der Formel I, dadurch gekennzeichnet, daß man

a) eine Verbindung der Formel II

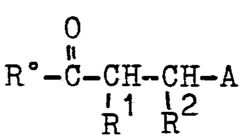

(II),

worin

A für eine nucleophil verdrängbare Abgangsgruppe wie Halogen, Tosyl, Mesyl oder eine tertiäre Alkylaminogruppe steht, mit einem Amin der Formel III,

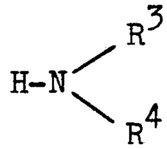

(III)

oder

b) eine Verbindung der Formel IV,

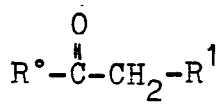

(IV)

mit einem Aldehyd der Formel V

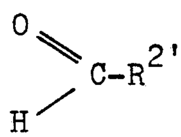

(V),

worin

$R^{2'}$ = H oder ($C_1$-$C_4$)-Alkyl bedeutet, und einem Amin der Formel III oder dessen Salz, insbesondere dessen Hydrochlorid oder Hydrobromid, oder

c) eine Verbindung der Formel VI,

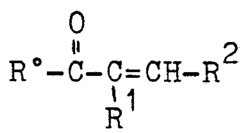

(VI)

mit einem Amin der Formel III oder dessen Salz, insbesondere dessen Hydrochlorid und Hydrobromid, oder

d) eine Verbindung der Formel VII

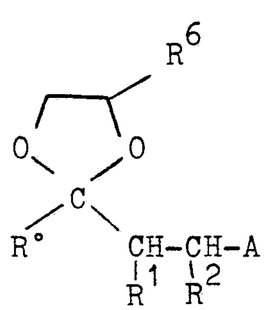

(VII)

mit einem Amin der Formel III umsetzt,

oder

die erhaltenen Verbindungen der Formel I mit X = CO , >CHOH, >C=NOH nach üblichen Methoden derivatisiert.

Die Verfahrensvarianten a) bis d) stellen für den Fachmann übliche Methoden zur Herstellung von tertiären Aminen und ihren Säureadditionssalzen dar und werden in an sich bekannter Weise durchgeführt:

Variante a): s. DE-OS 28 42 759; US-PS 3,426,036;

Variante b): s. DE-OS 30 19 497; DE-OS 30 34 383;

Variante c): s. DE-PS 1 768 500; BE-PS 733 213;

Variante d): s. US-PS 592,263.

Zur Derivatisierung werden beispielsweise Verbindungen der Formel I, in denen X = >CO bedeutet, mit katalytisch angeregtem Wasserstoff, komplexen Hydriden wie z.B. $LiAlH_4$, $LiBH_4$, $NaBH_4$ oder $NaHB(OCH_3)_3$ oder mit $Al[OCH(CH_3)_2]_3$ reduziert (DE-OS 30 34 383), oder nach üblichen Methoden in die Oxime, Semicarbazone oder Thiosemicarbazone überführt (DE-OS 30 19 497, DD-PS 143 427, SU-PS 229 498) oder mit Diolen in die cyclischen Acetale umgesetzt (Synthesis 1979 538).

Verbindungen der Formel I, in denen X = CHOH oder C=NOH bedeutet, können mit Alkylhalogeniden in bekannter Weise alkyliert werden (DE-OS 30 34 383, DE-OS 30 19 497).

Die Verbindungen der Formel I zeichnen sich durch eine hervorragende fungizide Wirksamkeit mit hoher Wirkungsbreite aus. Auch bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener, wirtschaftlich wichtiger phytopathogener Pilze, wie z.B. Piricularia oryzae und Pellicularia sasakii an Reis, verschiedene Rostarten, Venturia inaequalis am Apfel, Cercospora-Arten an Zuckerrübe, Soja und Erdnuß, Echte Mehltaupilze im Obst-, Gemüse-, Getreide-und Zierpflanzenbau sowie Botrytis cinerea am Wein. Darüber hinaus besitzen die Substanzen der Formel (I) hervorragende fungizide Eigenschaften gegenüber Phycomyceten, insbesondere Plasmopara viticola und Phytophthora infestans.

Die Verbindungen der Formel I eignen sich auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr-und Schneidölen.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs-oder Inertstoff noch Netzmittel, z.B. polyoxethylierte Alkylphenole, polyoxethylierte Fettalkohole, Alkyl-oder Alkylphenylsulfonate und Dispergiermittel, z.B. ligninsulfonsaures Natrium, 2,2'-disulfonsaures Natrium, dibutylnaphthalinsulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten, organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:

Alkylarylsulfonsaure Calciumsalze wie Ca-dodecylbenzolsulfonat oder nichtionische Emulgatoren wie Fettsäurepolyglycolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitan-fettsäureester oder Polyoxethylen-sorbitester.

Stäubemittel kann man durch Vermahlen des Wirkstoffes mit fein verteilten, festen Stoffen, z.B. Talkum natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinit oder von granuliertem Inertmaterial. Auch können geeignete Wirkstoffe in der für die Herstellung von Düngemittelgranulaten üblichen Weise - gewünschtenfalls in Mischung mit Düngemitteln -granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel-, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die Aufwandmengen an Wirkstoff der Formel I können in weiten Grenzen variieren und liegen in der fertigen Spritzbrühe im allgemeinen zwischen 1 mg und 500 mg Wirkstoff/Liter, oder zwischen 100 bis 1500 g Wirkstoff/ha. Die Pflanzen werden bei der Anwendung tropfnaß gespritzt.

A. Formulierungsbeispiele

Ein Stäubemittel wird erhalten, indem man z.B. a) 10 Gewichtsteile Wirkstoff mit 90 Gewichtsteilen Talkum als Inertstoff mischt und in einer Schlagmühle zerkleinert oder b) 60 Gewichtsteile Wirkstoff, 35 Gewichtsteile Talkum-Pulver und 5 Gewichtsteile Haftmittel (z. B. ein Polysaccharid wie ®Rhodopol) in der gleichen Weise homogenisiert.

Ein in Wasser leicht dispergierbares, benetzbares Pulver wird durch Mahlen einer Mischung aus 25 Gewichtsteilen Wirkstoff, 64 Gewichtsteilen kaolinhaltigem Quarz als Inertstoff, 10 Gewichtsteilen ligninsulfonsaurem Kalium und 1 Gewichtsteil oleoylmethyltaurinsaurem Natrium als Netz- und Dispergiermittel in einer Stiftmühle erhalten.

Eine Formulierung mit 5 Gew.-% Wirkstoffgehalt kann z.B. wie folgt zusammengesetzt sein: 5 Gew.-% Wirkstoff, 6% eines sulfonierten Naphthalinformaldehydkonzentrats - (z.B. ®Dispersogen A), 2 % eines Na-Salzes einer Alkylnaphthalinsulfonsäure (z. B. ® Leonil DB), 5% eines Gemisches aus Polypropylenglykol und $SiO_2$ (z. B. ®Acrotin 341), 25 % einer Kieselsäure (z. B. ®Sipernat) und 57 % Kaolin Typ 1777 und wird wie oben angegeben hergestellt.

Ein in Wasser leicht dispergierbares Dispersionskonzentrat stellt man her, indem man 20 Gewichtsteile Wirkstoff mit 6 Gewichtsteilen Alkylphenolpolyglykolether - (®Triton X 207), 3 Gewichtsteilen Isotridecanolpolyglykolether (8 EO) und 71 Gewichtsteilen paraffinischem Mineralöl (Siederbereich z.B. ca. 255 bis über 377°C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertem Nonylphenol (10 AeO) als Emulgator.

B. HERSTELLUNGSBEISPIELE

Beispiel 1

3-(Hexahydro-1H-azepin-1-yl)-4'-phenoxypropiophenon-hydrochlorid

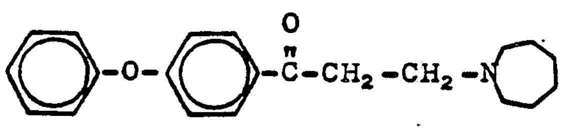

x HCl

13,0 g (0.05 mol) ω-Chlor-4-phenoxypropiophenon werden in 80 ml Aceton gelöst und bei Umgebungstemperatur tropfenweise mit einer Lösung von 5,0 g (0.05 mol) Hexamethylenimin in 20 ml Aceton versetzt. Nach Abklingen der exothermen Reaktion rührt man noch 3 h bei Raumtemperatur nach, saugt das kristalline Produkt ab, wäscht mit Aceton und trocknet das Produkt im Vakuum-Trockenschrank. Man erhält 15,8 g (88 % d.Th.) farbloses 3-(Hexahydro-1H-azepin-1-yl)-4'-phenoxypropiophenonhydrochlorid mit einem Schmelzpunkt von 153-155°C.

Beispiel 2

3-(Hexahydro-1H-azepin-1-yl)-3'-nitropropiophenonhydrochlorid

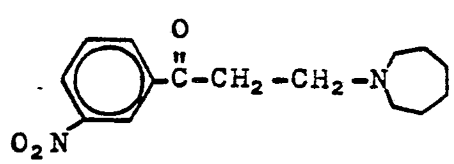

x HCl

16,5 g (0.1 mol) 3-Nitroacetophenon werden zusammen mit 14,0 g (0.103 mol) Hexamethyleniminhydrochlorid und 4,0 g (0.045 mol) Paraformaldehyd in 20 ml 96 %-igem Ethanol nach Zugabe von 0,2 ml konzentrierter HCl 2 Stunden auf Rückflußtemperatur erwärmt. Die noch warme Reaktionslösung wird in 100 ml Aceton eingetragen. Man beläßt die Mischung über Nacht im Eisschrank, saugt das kristalline Produkt ab, wächst mit Aceton und trocknet das Produkt im Vakuumtrockenschrank. Man erhält 25,8 g (83 % d.Th.). farbloses 3-(Hexahydro-1H-azepin-1-yl)-3'-nitropropiophenonhydrochlorid mit einem Schmelzpunkt von 172-174°C.

Beispiel 3

3-N,N-Dimethylamino-4'-phenoxypropiophenonhydrochlorid

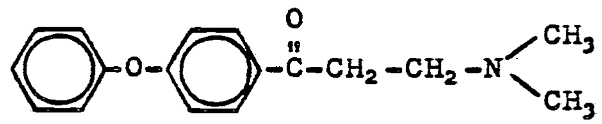

x HCl

Zu einer Lösung von 16,3 g (0,2 mol) Dimethylaminhydrochlorid in 15 ml 30-40 %iger wäßriger Formaldehydlösung gibt man 20 ml Acetanhydrid und rührt die Mischung zunächst 30 Minuten bei Umgebungstemperatur, - schließlich nochmals 30 Minuten bei 120°C. Danach fügt man eine siedend-heiße Lösung von 42,4 g (0.2 mol) 4-Phenoxyacetophenon in 30 ml Acetanhydrid hinzu und beläßt das Gemisch noch 15 Minuten bei Rückflußtemperatur. Nach Erkalten wird das Reaktionsgemisch im Wasserstrahlvakuum eingeengt und der verbleibende Rückstand in 100 ml Aceton aufgenommen und durch Anreiben zur Kristallisation gebracht. Nach Absaugen und Waschen mit Aceton wird das kristalline Produkt im Vakuum getrocknet. Man erhält 30,4 g ( 50 % d.Th.) farbloses 3-N,N-Dimethylamino-4'-phenoxypropiophenonhydrochlorid mit einem Schmelzpunkt von 149-151 °C.

Beispiel 4

3-(Hexahydro-1H-azepin-1-yl)-4'-phenoxypropiophenon

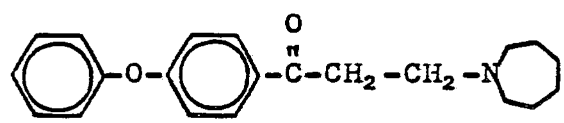

36 g (0.1 mol) gemäß Beispiel 1 hergestelltes 3-(Hexahydro-1H-azepin-1-yl)-4'-phenoxypropiophenonhydrochlorid werden in 300 ml Methylenchlorid gelöst und 2 mal mit je 50 ml gesättigter Natriumhydrogencarbonatlösung intensiv ausgeschüttelt. Danach wäscht man die organische Phase noch 1 mal mit 100 ml Wasser, trocknet sie über $Na_2SO_4$ und entfernt schließlich das Lösungsmittel im Wasserstrahlvakuum. Der verbleibende ölige Rückstand wird im Oelpumpenvakuum getrocknet. Man erhält 31 g (97 % d.Th.) 3-Hexahydro-1H-azepin-1-yl)-4'-phenoxypropiophenon in Form eines zähflüssigen, gelben Oels mit einem Brechungsindex von $n_D^{20}$ -1,5817.

Beispiel 5

3-(Hexahydro-1H-azepin-1-yl)-1-(4-phenoxyphenyl)-propanol

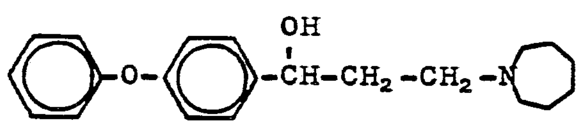

18 g (0.05 mol) gemäß Beispiel 1 hergestelltes 3-(Hexahydro-1H-azepin-1-yl)-4'-phenoxypropiophenonhydrochlorid werden bei Umgebungstemperatur in 80 ml 50 %igem Ethanol suspendiert und unter Rühren mit einer Lösung von 2,1 g (0,053 mol) NaOH in 120 ml 96 %igem Ethanol versetzt. Zu der klaren Lösung fügt man schließlich 1,95 g (0.05 mol) 96 %iges $NaBH_4$ hinzu und läßt noch 30 Minuten bei Raumtemperatur rühren. Anschließend versetzt man die Reaktionslösung mit 600 ml $CH_2Cl_2$, wäscht 3 mal sorgfältigst mit je 100 ml $H_2O$ und trocknet den $CH_2Cl_2$-Extrakt über $Na_2SO_4$. Danach entfernt man das Lösungsmittel im Wasserstrahlvakuum und trocknet den verbleibenden Rückstand im Oelpumpenvakuum. Man erhält 15,6 g 91 %iges 3-(Hexahydro-1H-azepin-1-yl)-1-(4-phenoxyphenyl)propanol in Form eines dickflüssigen gelben Oels, das sich durch Kugelrohrdestillation weiter reinigen läßt. Reinheit nach Kugelrohrdestillation: 95 %ig (gaschromatographisch).

Beispiel 6

3-(Hexahydro-1H-azepin-1-yl)-1-(4-phenoxyphenyl)propanol-hydrochlorid

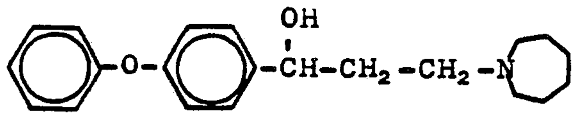

x HCl

12,4 g (0.04 mol) 95 %iges 3-(Hexahydro-1H-azepin-1-yl)-1-(4-phenoxyphenyl)propanol (siehe Beispiel 5) werden in 100 ml iso-Propanol gelöst. Danach leitet man unter Rühren und Eiskühlung innerhalb von 60 Minuten 2,5 -3.0 g Chlorwasserstoff ein, rührt anschließend noch 15 Minuten bei Raumtemperatur nach und entfernt das Lösungsmittel im Wasserstrahlvakuum. Den verbleibenden Rückstand nimmt man in 20-30 ml Aceton auf, saugt den kristallinen Niederschlag ab, wäscht mit Aceton und trocknet das kristalline Produkt im Vakuum. Man erhält 10,3 g (79 % d.Th.) farbloses 3-(Hexahydro-1H-azepin-1-yl)-1-(4-phenoxyphenyl)propanolhydrochlorid mit einem Schmelzpunkt von 137-138°C.

Beispiel 7

1-[3-(2,4-Dichlorbenzyloxy)-3-(4-phenoxy)phenyl]propyl-hexahydro-1H-azepin

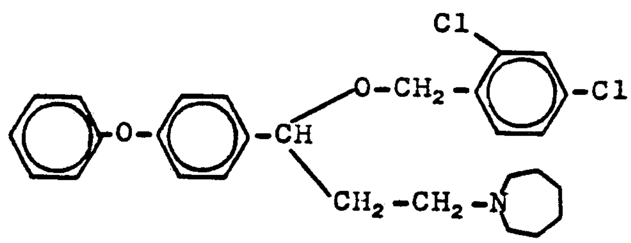

22,8 g (0.07 mol) gemäß Beispiel 5 hergestelltes 95 %iges 3-(Hexahydro-1H-azepin-1-yl)-1-(4-phenoxyphenyl)-propanol werden in 70 ml DMF gelöst und portionsweise mit 3 g (0.1 mol). 80 %igem NaH versetzt. Nach Zugabe wird noch 30 Minuten bei 40°C nachgerührt. Anschließend tropft man 15,l g (0.077 mol) 2,4-Dichlorbenzylchlorid zu. Hierbei steigt die Innentemperatur auf etwa 60°C an. Man erwärmt noch 4 Stunden auf 80°C, gießt die erkaltete Reaktionslösung auf 500 g Eis und extrahiert 3 mal mit je 100 ml $CH_2Cl_2$. Nach Trocknen über $Na_2SO_4$ entfernt man das Lösungsmittel im Wasserstrahlvakuum und reinigt das ölige Rohprodukt durch Säulenchromatographie an Kieselgel mit Methanol als Eluenten. Man erhält 20 g (53 % d.Th.) 90 %iges (NMR) gelbes 1-[3-(2,4-Dichlorbenzyloxy)-3-(4-phenoxy)phenyl]propyl-hexahydro-1H-azepin in Form eines stark viskosen Oels.

Beispiel 8

3-(Hexahydro-1H-azepin-1-yl)-4'-phenoxypropiophenonoxim

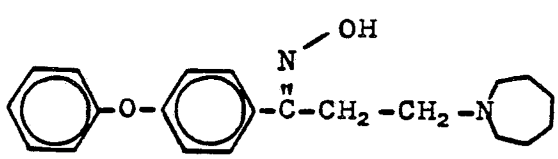

32,3 g (0.1 mol) gemäß Beispiel 4 hergestelltes 3-(Hexahydro-1H-azepin-1-yl)-4'-phenoxypropiophenon werden zusammen mit 20 g (0.19 mol) Natriumcarbonat und 14 g (0.2 mol) Hydroxylaminhydrochlorid in 80 ml 96 %igem Ethylalkohol 4 Stunden auf Rückflußtemperatur erwärmt. Nach Erkalten gießt man die Reaktions mischung auf etwa 500 g Eis und extrahiert das Gemisch 3 mal mit je 100 ml Essigsäurethylester. Die vereinigten Extrakte trocknet man über $Na_2SO_4$ und entfernt das Lösungsmittel anschließend im Wasserstrahlvakuum. Aus dem bräunlichgelben Rohprodukt erhält man nach Zugabe von Diethylether und Anreiben 12,5 g (37 % d.Th.) farbloses 3-(Hexahydro-1H-azepin-1-yl)-4'-phenoxypropiophenonoxim mit einem Schmelzpunkt von 140-142°C.

Beispiel 9

3-(Hexahydro-1H-azepin-1-yl)-4'-phenoxypropiophenonoxim-hydrochlorid

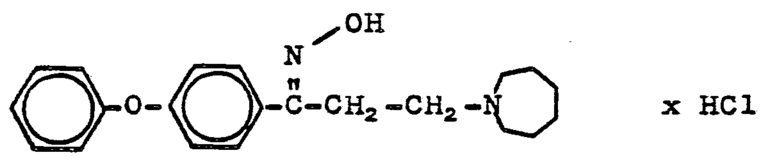

35,9 g (0.1 mol) gemäß Beispiel 1 hergestelltes 3-(Hexahydro-1H-azepin-1-yl)-4'-phenoxypropiophenonhydrochlorid werden zusammen mit 16,4 g (0.2 mol) wasserfreiem Natriumacetat und 14,0 g (0.2 mol) Hydroxylaminhydrochlorid 4-5 Stunden auf 80°C erwärmt. Die erkaltete Reaktionsmischung wird filtriert, der Filtrationsrückstand gut mit Ethylalkohol gewaschen und das Filtrat schließlich im Wasserstrahlvakuum eingeengt. Nach Aufnahme in Ether oder Aceton kristallisieren 20,3 g farbloses 3-(Hexahydro-1H-azepin-1-yl)-4'-phenoxypropiophenonoxim-hydrochlorid mit einem Schmelzpunkt von 172-174°C. Analysenreines Produkt erhält man, wenn man das erhaltene Hydrochlorid mehr fach in einem Gemisch von 300 ml $CH_2Cl_2$ und 100 ml $H_2O$ ausschüttelt, die $CH_2Cl_2$-Phasen vereinigt, über $Na_2SO_4$ trocknet und das Lösungsmittel schließlich im Wasserstrahlvakuum entfernt. Das so gereinigte Produkt zeigt einen Schmelzpunkt von 186-188°C.

Beispiele 10 bis 195

Analog zu den Beispielen 1 bis 9 können die in der nachfolgenden Tabelle 1 aufgeführten Verbindungen der Formel I hergestellt werden.

TABELLE 1

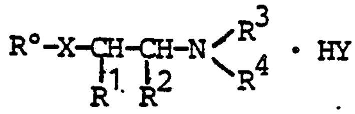

| Nr. | R° | $R^1$ | $R^2$ | X | $NR^3R^4$ | HY | Fp. [°C] |
|---|---|---|---|---|---|---|---|
| 10 | 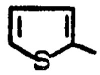 | H | H | CO | $N(CH_3)_2$ | HCl | 175-177 |
| 11 | 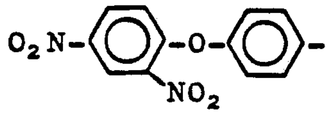 | H | H | CO | $N(CH_3)_2$ | HCl | 181-184 |
| 12 | 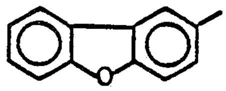 | H | H | CO | $N(CH_3)_2$ | HCl | 188-190 |
| 13 | 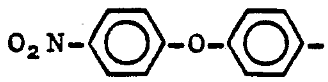 | H | H | CO | $N(CH_3)_2$ | HCl | 173-174 |
| 14 | 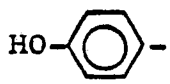 | H | H | CO | $N(CH_3)_2$ | HCl | 195-196 |
| 15 | 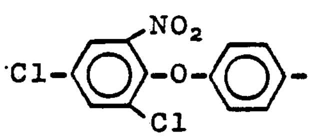 | H | H | CO | $N(CH_3)_2$ | HCl | 154-157 |
| 16 | 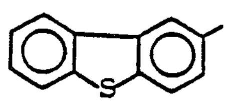 | H | H | CO | $N(CH_3)_2$ | HCl | 199-200 |
| 17 | 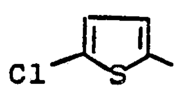 | H | H | CO | 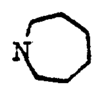 | HCl | 154-155 |
| 18 | 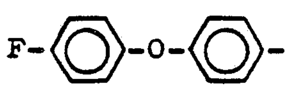 | H | H | CO | 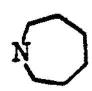 | HCl | 158-160 |
| 19 | 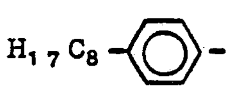 | H | H | CO | 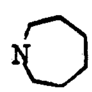 | HCl | 145-146 |
| 20 | 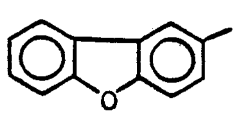 | H | H | CO | 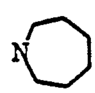 | HCl | 185-188 |
| 21 | 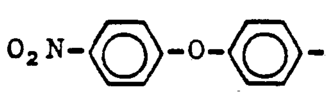 | H | H | CO | 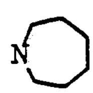 | HCl | 166-169 |

Forts. Tabelle 1

| Nr. | R° | $R^1$ | $R^2$ | X | $NR^3R^4$ | HY | Fp. [°C] |
|---|---|---|---|---|---|---|---|
| 22 | 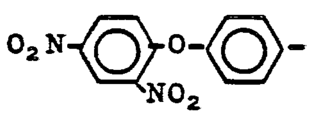 | H | H | CO | 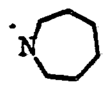 | HCl | 174-176 |
| 23 | 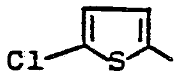 | H | H | CO | 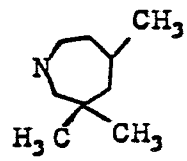 | HCl | 148-149 |
| 24 | 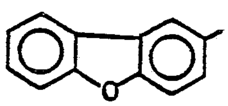 | H | H | CO | 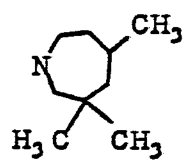 | HCl | 154-158 |
| 25 | 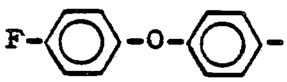 | H | H | CO | 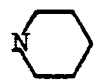 | HCl | 173-174 |
| 26 | 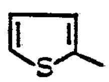 | H | H | CO | 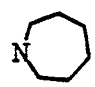 | HCl | 150-151 |
| 27 | 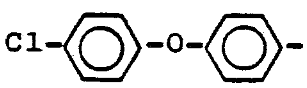 | H | H | CO | 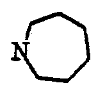 | HCl | 165-167 |
| 28 | 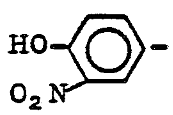 | H | H | CO | 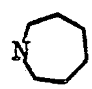 | HCl | 157-159 |
| 29 | 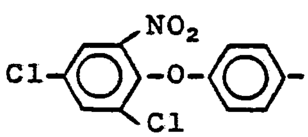 | H | H | CO | 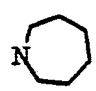 | HCl | 185-187 |
| 30 | 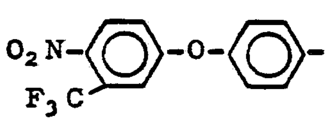 | H | H | CO | 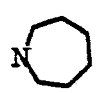 | HCl | 190-192 |
| 31 | 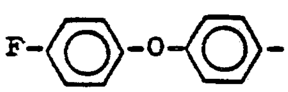 | H | H | CO | 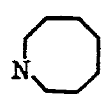 | HCl | 145-147 |
| 32 | 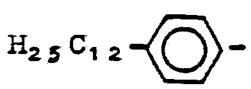 | H | H | CO | 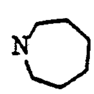 | HCl | 138-141 |

Forts. Tabelle 1

| Nr. | R° | $R^1$ | $R^2$ | X | $NR^3R^4$ | HY | Fp. [°C] |
|---|---|---|---|---|---|---|---|
| 33 | 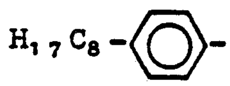 | H | H | CO | 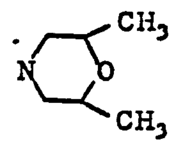 | HCl | 190-192 |
| 34 | 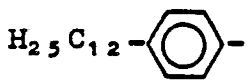 | H | H | CO | 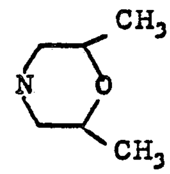 | HCl | 178-180 |
| 35 | 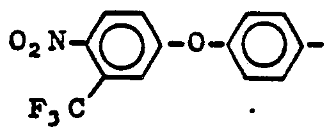 | H | H | CO | 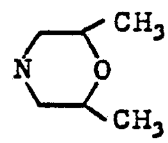 | HCl | 214-217 |
| 36 | 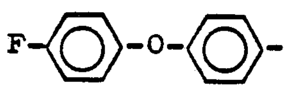 | H | H | CO | 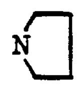 | HCl | 164-166 |
| 37 | 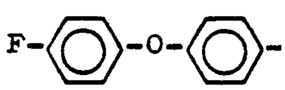 | H | H | CO | 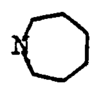 | - | Oel |
| 38 | 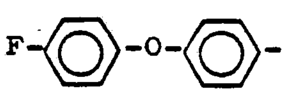 | H | H | CO | 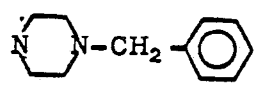 | HCl | 170-172 |
| 39 | 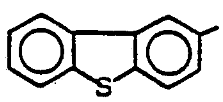 | H | H | CO | 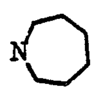 | HCl | 180-181 |
| 40 | 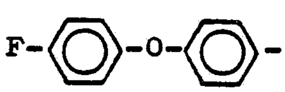 | H | H | CO | $N(C_2H_5)_2$ | HCl | 118-122 |
| 41 | 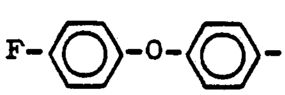 | H | H | CO | 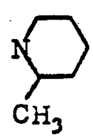 | HCl | 138-140 |
| 42 | 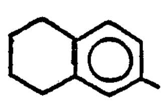 | H | H | CO | 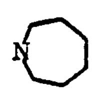 | HCl | 162-164 |
| 43 | 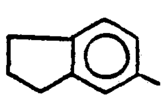 | H | H | CO | 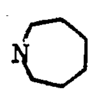 | HCl | 162-164 |

Forts. Tabelle 1

| Nr. | R° | $R^1$ | $R^2$ | X | $NR^3R^4$ | HY | Fp. [°C] |
|---|---|---|---|---|---|---|---|
| 44 | 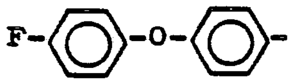 | H | H | CO | $N(C_4H_9)_2$ | HCl | 147-149 |
| 45 | 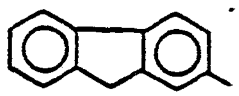 | H | H | CO | 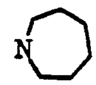 | HCl | 199-200 |
| 46 | 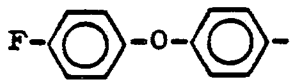 | H | H | CO | 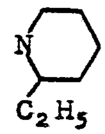 | HCl | 123-126 |
| 47 | 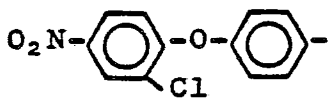 | H | H | CO | 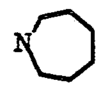 | HCl | 161-163 |
| 48 | 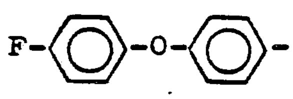 | H | H | CO | 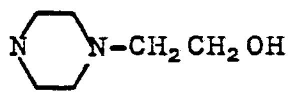 | HCl | 155-157 |
| 49 | 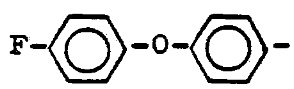 | H | H | CO | 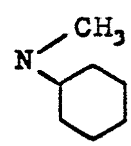 | HCl | 139-147 |
| 50 | 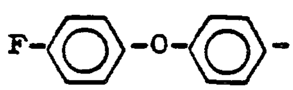 | H | H | CO | 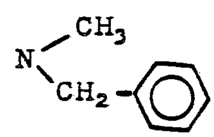 | HCl | 119-122 |
| 51 | 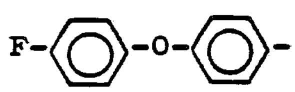 | H | H | CO | 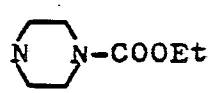 | HCl | 176-177 |
| 52 | 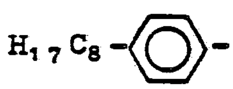 | H | H | CO | 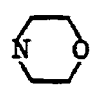 | HCl | 154-156 |
| 53 | 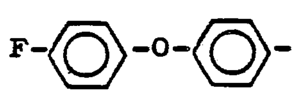 | H | H | CO | 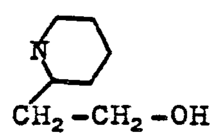 | HCl | 131-133 |
| 54 | 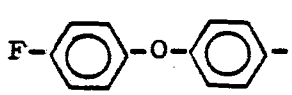 | H | H | CO | 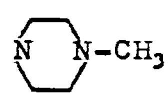 | HCl | 156-162 |

Forts. Tabelle 1

| Nr. | R° | $R^1$ | $R^2$ | X | $NR^3R^4$ | HY | Fp. [°C] |
|---|---|---|---|---|---|---|---|
| 55 | 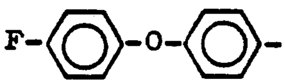 | H | H | CO | 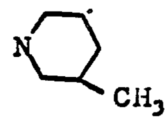 | HCl | 188-189 |
| 56 | 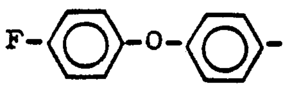 | H | H | CO | 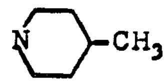 | HCl | 181-183 |
| 57 | 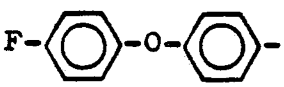 | H | H | CO | 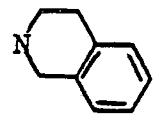 | HCl | 188-190 |
| 58 | 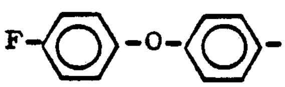 | H | H | CO | 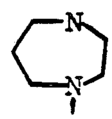 $CHR^2CHR^1XR°$ 1) | 2 HCl | 164-165 |
| 59 | 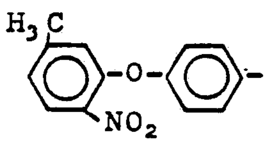 | H | H | CO | 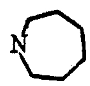 | HCl | 168-169 |
| 60 | 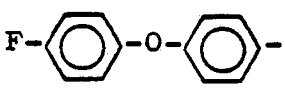 | H | H | CO | 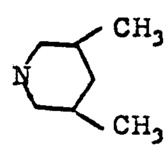 | HCl | 157-158 |
| 61 | 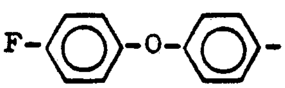 | H | H | CO | 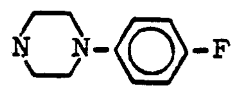 | HCl | 189-190 |
| 62 | 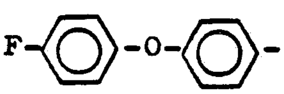 | H | H | CO | 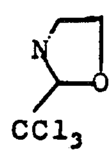 | - | 76-78 |
| 63 | 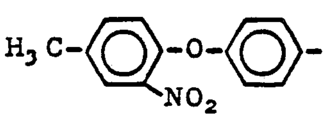 | H | H | CO | 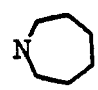 | HCl | 158-159 |
| 64 | 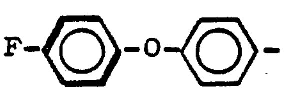 | H | H | CO | $N(CH_2CH_2OH)_2$ | HCl | 124-125 |

1) $R^2$, $R^1$, X und R° besitzt die nebenstehende Bedeutung

Forts. Tabelle 1

| Nr. | R° | $R^1$ | $R^2$ | X | $NR^3R^4$ | HY | Fp. [°C] |
|---|---|---|---|---|---|---|---|
| 65 | 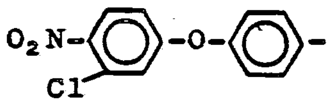 | H | H | CO | 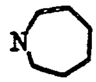 | HCl | 165-166 |
| 66 | 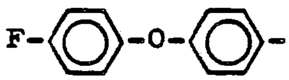 | H | H | CO | 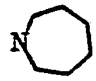 | HBr | 169-170 |
| 67 | 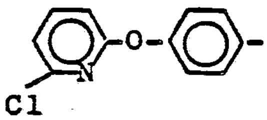 | H | H | CO | $N(CH_3)_2$ | HCl | 188-189 |
| 68 | 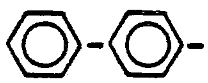 | H | H | CO | 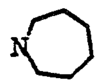 | HCl | 176-177 |
| 69 | 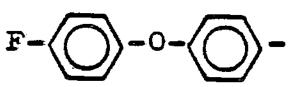 | H | H | CO | 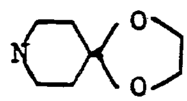 | HCl | 170-175 |
| 70 | 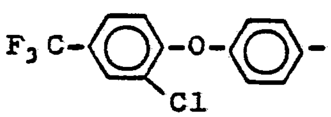 | H | H | CO | $N(CH_3)_2$ | HCl | 141-144 |
| 71 | 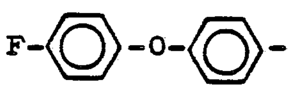 | $CH_3$ | H | CO | 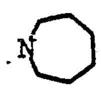 | HCl | 165 |
| 72 | 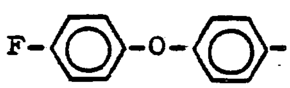 | H | H | CHOH | 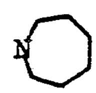 | - | 76-78 |
| 73 | 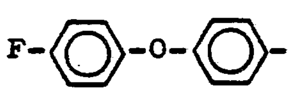 | H | H | CO | 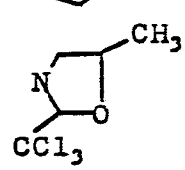 | - | Oel* |
| 74 | 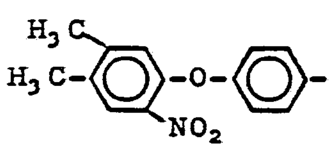 | H | H | CO | 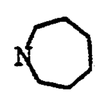 | HCl | 194-195 |
| 75 | 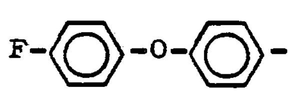 | $CH_3$ | H | CO | $N(CH_3)_2$ | HCl | 187-188 |

Forts. Tabelle 1

| Nr. | R° | $R^1$ | $R^2$ | X | $NR^3R^4$ | HY | Fp. [°C] |
|---|---|---|---|---|---|---|---|
| 76 | 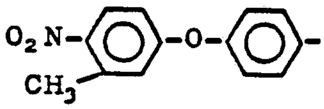 | H | H | CO | 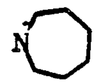 | HCl | 183-184 |
| 77 | 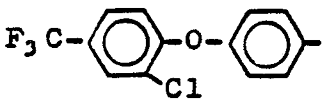 | H | H | CO | 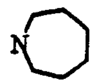 | HCl | 170-173 |
| 78 | 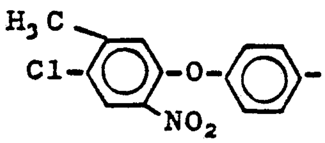 | H | H | CO | 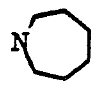 | HCl | 221-224 |
| 79 | 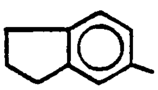 | H | H | CHOH | 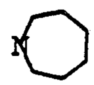 | - | Oel |
| 80 | 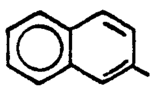 | H | H | CO | 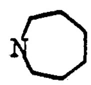 | HCl | 160-165 |
| 81 | 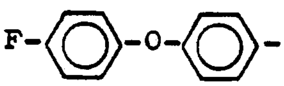 | H | H | CO | 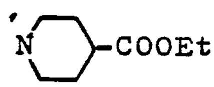 | HCl | 154-156 |
| 82 | 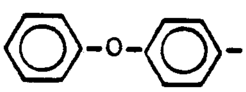 | H | H | CO | 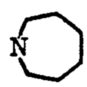 | 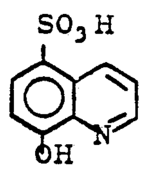 | 127-130 |
| 83 | 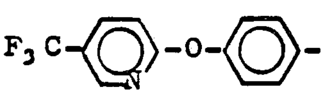 | H | H | CO | $N(CH_3)_2$ | HCl | 190-192 |
| 84 | 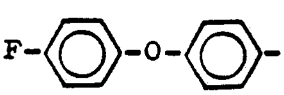 | H | H | CO | 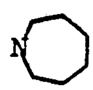 | 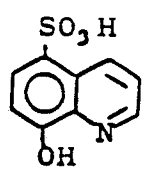 | 122-123 |
| 85 | 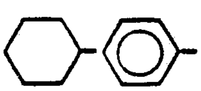 | H | H | CHOH | 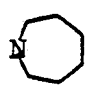 | - | Oel |

Forts. Tabelle 1

| Nr. | R° | $R^1$ | $R^2$ | X | $NR^3R^4$ | HY | Fp. [°C] |
|---|---|---|---|---|---|---|---|
| 86 | 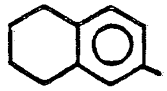 | H | H | CHOH | 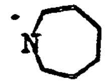 | - | Oel |
| 87 | 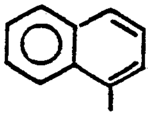 | H | H | CO | 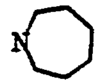 | HCl | 146-147 |
| 88 | 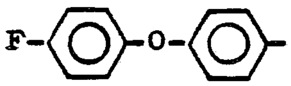 | H | H | CO | 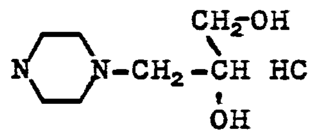 | HCl | 156-157 |
| 89 | 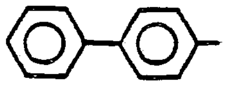 | H | H | CHOH | 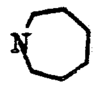 | - | 72-74 |
| 90 | 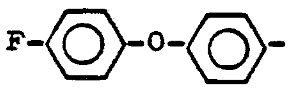 | H | H | CHOH | 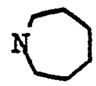 | HCl | 123-130 |
| 91 | 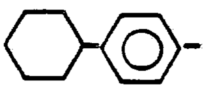 | H | H | CHOH | 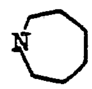 | HCl | 203-205 |
| 92 | 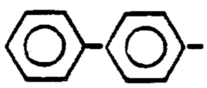 | H | H | CHOH | 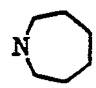 | HCl | 178-180 |
| 93 | 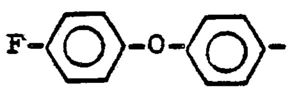 | H | H | CO | 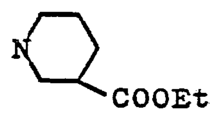 | HCl | 158-159 |
| 94 | 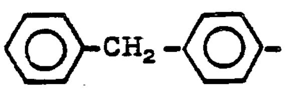 | H | H | CO | 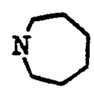 | HCl | 157-158 |
| 95 | 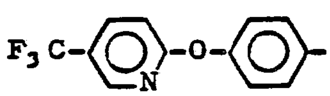 | H | H | CO | 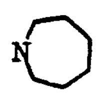 | HCl | 160 |
| 96 | 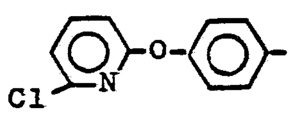 | H | H | CO | 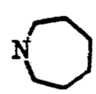 | HCl | 136-139 |
| 97 | 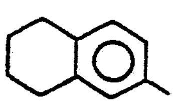 | H | H | CHOH | 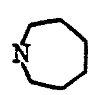 | HCl | 140-143 |

Forts. Tabelle 1

| Nr. | R° | $R^1$ | $R^2$ | X | $NR^3R^4$ | HY | Fp. [°C] |
|---|---|---|---|---|---|---|---|
| 98 | 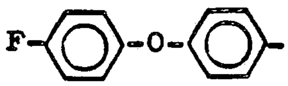 | $CH_3$ | H | CHOH | 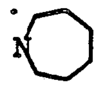 | HCl | 113-115 ** |
| 99 | 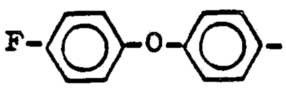 | H | H | CO | 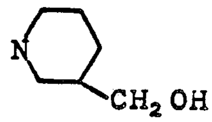 | HCl | 157-158 |
| 100 | 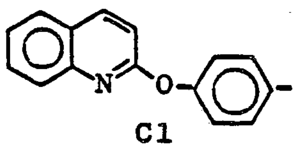 | H | H | CO | 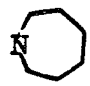 | HCl | 160-162 |
| 101 | 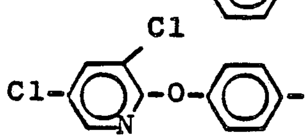 | H | H | CO | 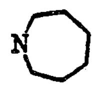 | HCl | 144-147 |
| 102 | 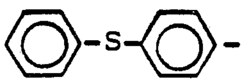 | H | H | CO | 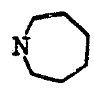 | HCl | 154 |
| 103 | 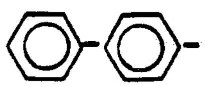 | H | H | CO | 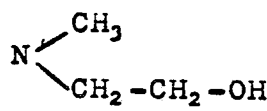 | HCl | 174-175 |
| 104 | 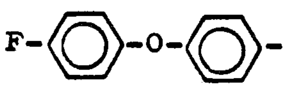 | H | H | CO | 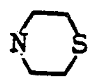 | HCl | 185-186 |
| 105 | 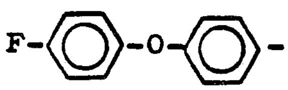 | H | H | CO | 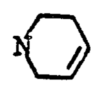 | HCl | 173-174 |
| 106 | 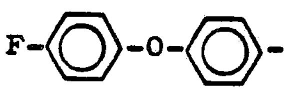 | H | H | CO | 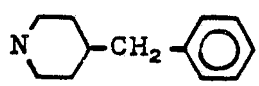 | HCl | 195-197 |
| 107 | 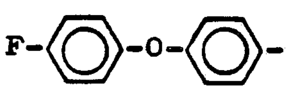 | H | H | CO | 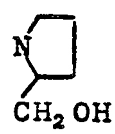 | HCl | 152-155 *** |
| 108 | 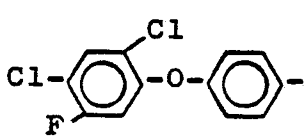 | H | H | CO | 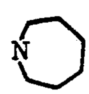 | HCl | 174-176 |

Forts. Tabelle 1

| Nr. | R° | $R^1$ | $R^2$ | X | $NR^3R^4$ | HY | Fp. [°C] |
|---|---|---|---|---|---|---|---|
| 109 | 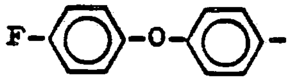 | H | H | CO | 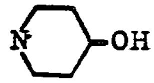 | HCl | 143-145 |
| 110 | 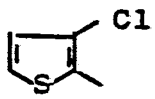 | H | H | CO | 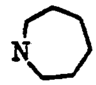 | HCl | 136-138 |
| 111 | 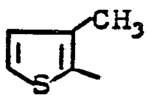 | H | H | CO | 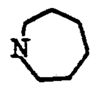 | HCl | 142-143 |
| 112 | 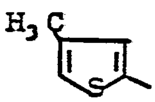 | H | H | CO | 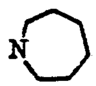 | HCl | 143-145 |
| 113 | 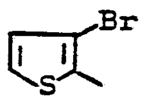 | H | H | CO | 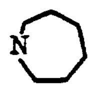 | HCl | 131-134 |
| 114 | $CF_3CHFCF_2O-$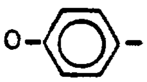 | H | H | CO | 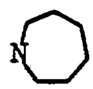 | HCl | 145-147 |
| 115 | 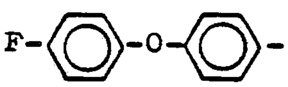 | H | H | CO | 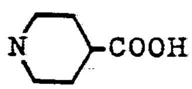 | HCl | > 250 |
| 116 | 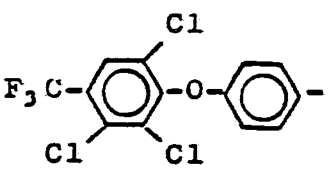 | H | H | CO | 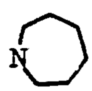 | HCl | 180 |
| 117 | 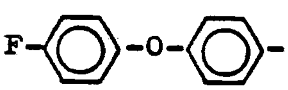 | H | H | CO | 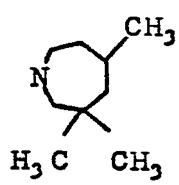 | HCl | 124-126 |
| 118 | 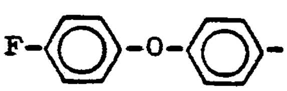 | H | H | CO | 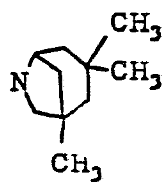 | HCl | Oel |

Forts. Tabelle 1

| Nr. | R° | $R^1$ | $R^2$ | X | $NR^3R^4$ | HY | Fp. [°C] |
|---|---|---|---|---|---|---|---|
| 119 | 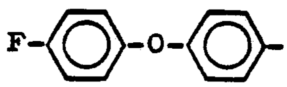 | H | H | CO | 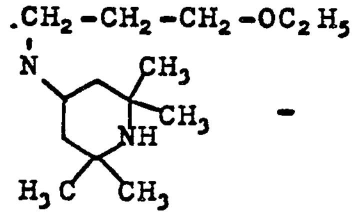 | - | Oel |
| 120 | 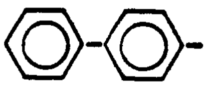 | H | H | CO | " | HCl | Oel |
| 121 | 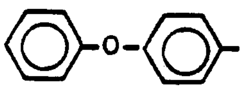 | H | H | $CHOCH_2$ 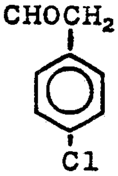 | 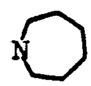 | - | Oel |
| 122 | 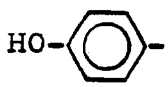 | H | H | CO | 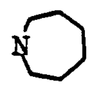 | HCl | 177-178 |
| 123 | 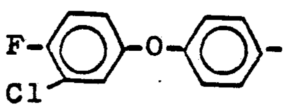 | H | H | CO | 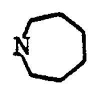 | HCl | 178-180 |
| 124 | 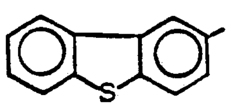 | H | H | CHOH | 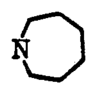 | HCl | 159-161 |
| 125 | 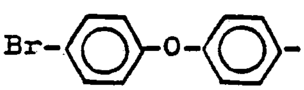 | H | H | CO | 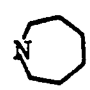 | HCl | 172 |
| 126 | 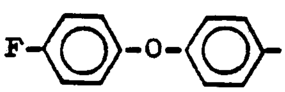 | H | H | CO | 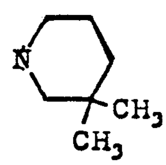 | HCl | 170 |
| 127 | 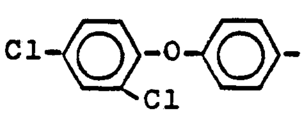 | H | H | CO | 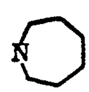 | HCl | 178-180 |

Forts. Tabelle 1

| Nr. | R° | $R^1$ | $R^2$ | X | $NR^3R^4$ | HY | Fp. [°C] |
|---|---|---|---|---|---|---|---|
| 128 | 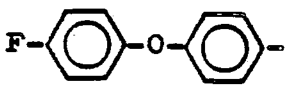 | H | H | CO | 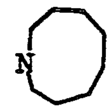 | HCl | 130-131 |
| 129 | 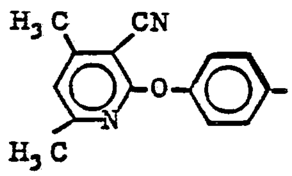 | H | H | CO | 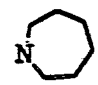 | HCl | 198-200 |
| 130 | 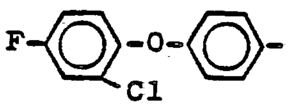 | H | H | CO | 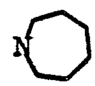 | HCl | 158-159 |
| 131 | 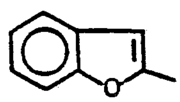 | H | H | CO | 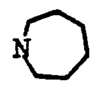 | HCl | 164 |
| 132 | 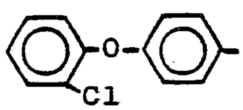 | H | H | CO | 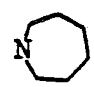 | HCl | 142-146 |
| 133 | 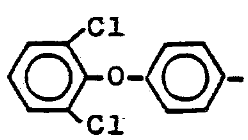 | H | H | CO | 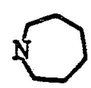 | HCl | 158-160 |
| 134 | 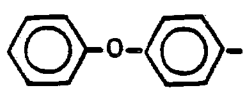 | H | H | $CHOCH_2$- 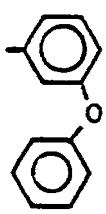 | 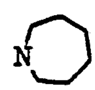 | - | Oel |
| 135 | 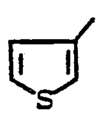 | H | H | CO | 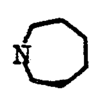 | HCl | 156-158 |
| 136 | 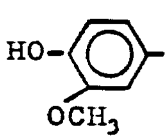 | H | H | CO | 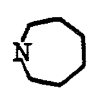 | HCl | 153-155 |

Forts. Tabelle 1

| Nr. | R° | $R^1$ | $R^2$ | X | $NR^3R^4$ | HY | Fp. [°C] |
|---|---|---|---|---|---|---|---|
| 137 | 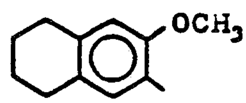 | H | H | CO | 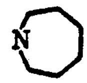 | HCl | 142-144 |
| 138 | 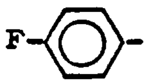 | $CH_2CH_2Cl$ | H | CO | 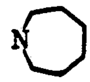 | HCl | 92-94 |
| 139 | 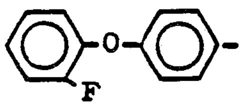 | H | H | CO | 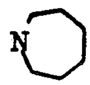 | HCl | 153-154 |
| 140 | 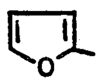 | H | H | CO | $N(CH_3)_2$ | HCl | 173-174 |
| 141 | 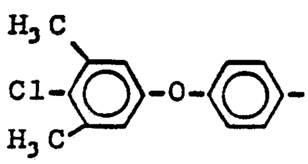 | H | H | CO | 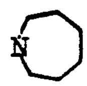 | HCl | 205-206 |
| 142 | 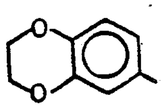 | H | H | CO | 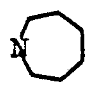 | HCl | 164 |
| 143 | 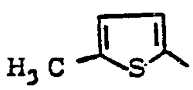 | H | H | CO | 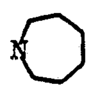 | HCl | 146-147 |
| 144 | 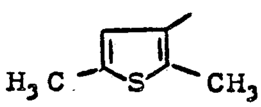 | H | H | CO | 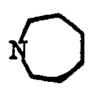 | HCl | 174-175 |

Forts. Tabelle 1

| Nr. | R° | $R^1$ | $R^2$ | X | $NR^3R^4$ | HY | Fp.[°C] |
|---|---|---|---|---|---|---|---|
| 145 | 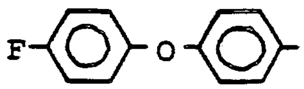 | H | H | CO | 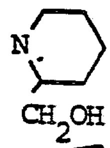 | HCl | 131-133 |
| 146 | 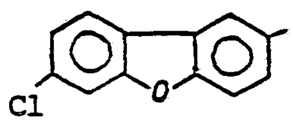 | H | H | CO | 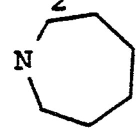 | HCl | |
| 147 | 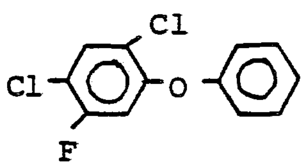 | H | H | CNOH | 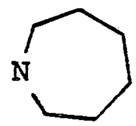 | HCl | 196-199 |
| 148 | 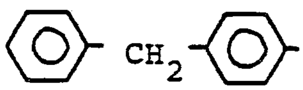 | H | H | CNOH | 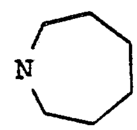 | HCl | 186 |
| 149 | 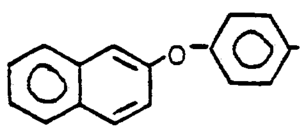 | H | H | CO | 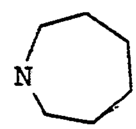 | HCl | |
| 150 | 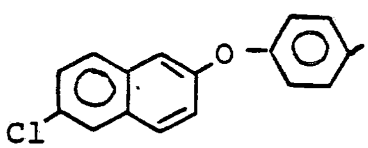 | H | H | CO | 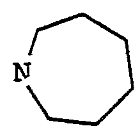 | HCl | |
| 151 | 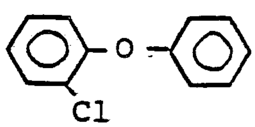 | H | H | CNOH | 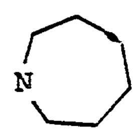 | HCl | |
| 152 | 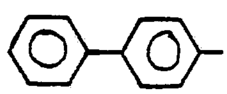 | H | H | CNOH | 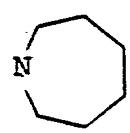 | HCl | 205-208 |
| 153 | 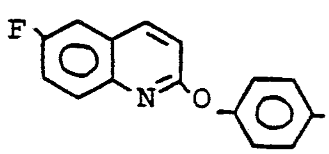 | H | H | CO | 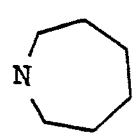 | HCl | |

Forts. Tabelle 1

| Nr. | R° | $R^1$ | $R^2$ | X | $NR^3R^4$ | HY | Fp. [°C] |
|---|---|---|---|---|---|---|---|
| 154 | 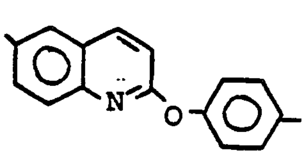 | H | H | CO | 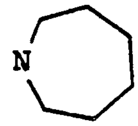 | HCl | |
| 155 | 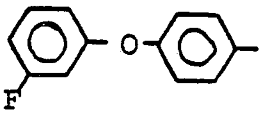 | H | H | CO | 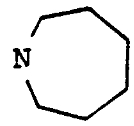 | HCl | |
| 156 | 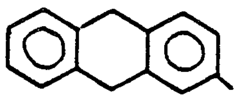 | H | H | CO | 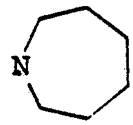 | HCl | 174-176 |
| 157 | 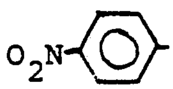 | H | H | CO | 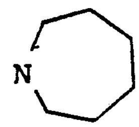 | HCl | |
| 158 | 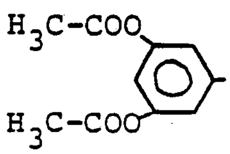 | H | H | CO | 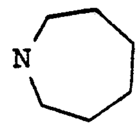 | HCl | 155-156 |
| 159 | 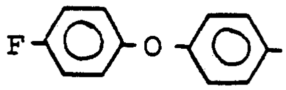 | H | H | CO | $N(CH_2CH{=}CH_2)_2$ | HCl | 130-132 |
| 160 | 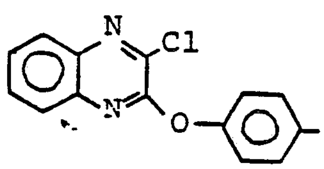 | H | H | CO | 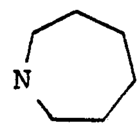 | HCl | |
| 161 | 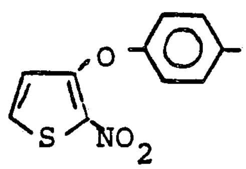 | H | H | CO | 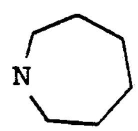 | HCl | |
| 162 | 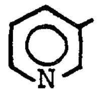 | H | H | CO | 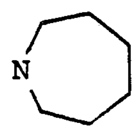 | HCl | |
| 163 | 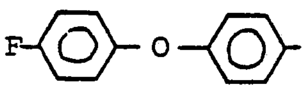 | H | H | CO | 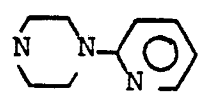 | HCl | 185-187 |
| 164 | 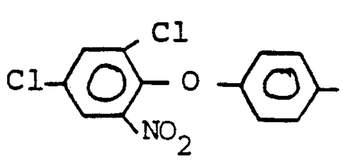 | H | H | CO | 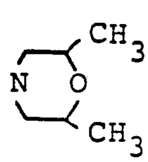 | HCl | 202-205 |

Forts. Tabelle 1

| Nr. | R° | $R^1$ | $R^2$ | X | $NR^3R^4$ | HY | Fp.[°C] |
|---|---|---|---|---|---|---|---|
| 165 | 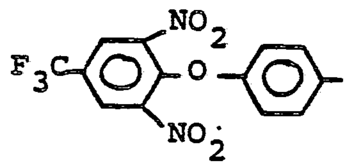 | H | H | CO | 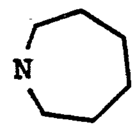 | HCl | |
| 166 | 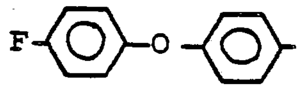 | H | H | CO | 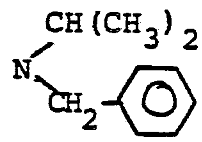 | HCl | 149-150 |
| 167 | 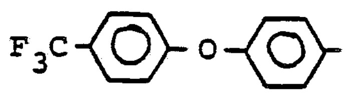 | H | H | CO | 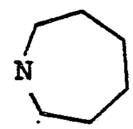 | HCl | |
| 168 | 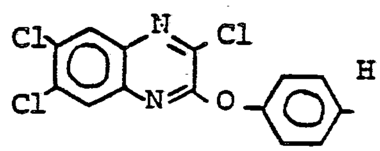 | H | H | CO | 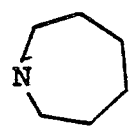 | HCl | |
| 169 | 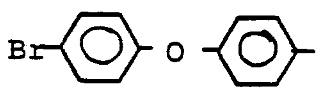 | H | H | CNOH | 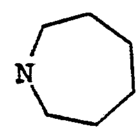 | HCl | 182-184 |
| 170 | 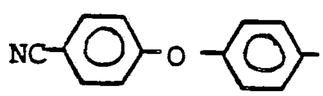 | H | H | CO | 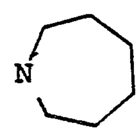 | HCl | |
| 171 | 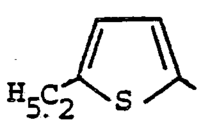 | H | H | CO | 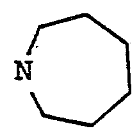 | HC | 154-156 |
| 172 | 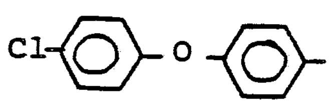 | H | H | CNOH | 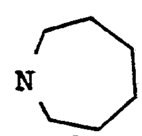 | HCl | 185-188 |
| 173 | 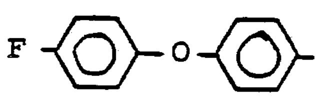 | H | H | CNOH | 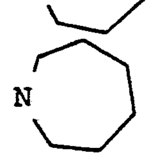 | HCl | |
| 174 | 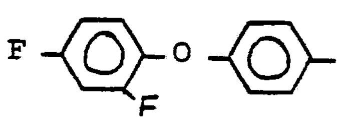 | H | H | CO | 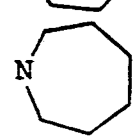 | HCl | |
| 175 | 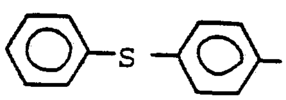 | H | H | CNOH | 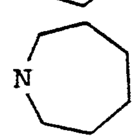 | HCl | 190-192 |

Forts. Tabelle 1

| Nr. | R° | $R^1$ | $R^2$ | X | $NR^3R^4$ | HY | Fp.[°C] |
|---|---|---|---|---|---|---|---|
| 176 | 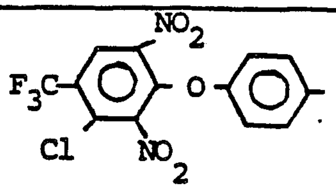 | H | H | CNOH | 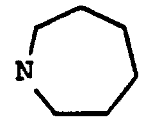 | HCl | |
| 177 | 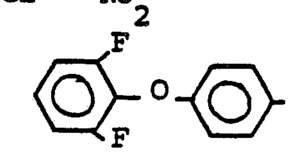 | H | H | CO | 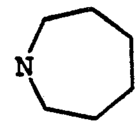 | HCl | |
| 178 | 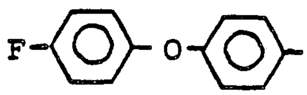 | H | H | CO | $N(CH_2COOH)_2$ | - | |
| 179 | 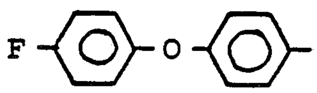 | H | H | CO | 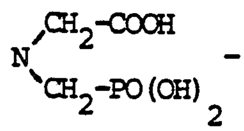 | - | |
| 180 | 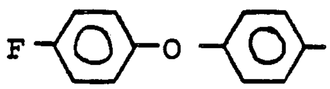 | H | H | CO | $N(CH_2CN)_2$ | HCl | |
| 181 | 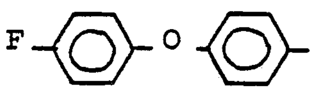 | H | H | CO | 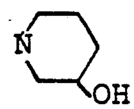 | HCl | |
| 182 | 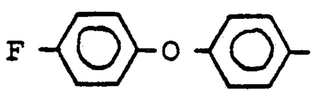 | H | H | CO | 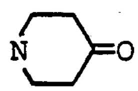 | HCl | |
| 183 | 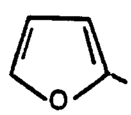 | H | H | CO | 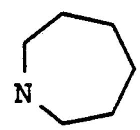 | HCl | 123-124 |
| 184 | 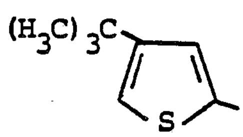 | H | H | CO | 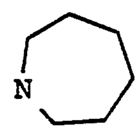 | HCl | |
| 185 | 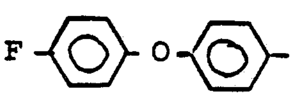 | H | H | CO | 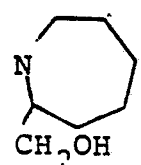 | HCl | |
| 186 | 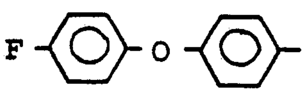 | H | H | CO | 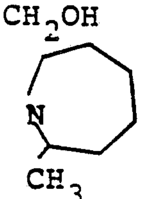 | HCl | |
| 187 | 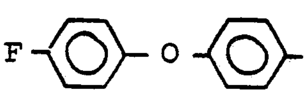 | H | H | CO | 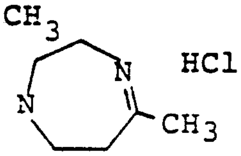 | HCl | |

Forts. Tabelle 1

| Nr. | R° | $R^1$ | $R^2$ | X | $NR^3R^4$ | HY | Fp. [°C] |
|---|---|---|---|---|---|---|---|
| 188 | 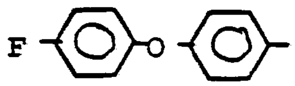 | H | H | CO | 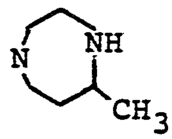 | HCl | |
| 189 | 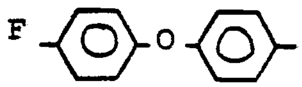 | H | H | CO | 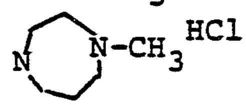 | HCl | |
| 190 | 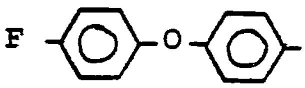 | H | H | CO | 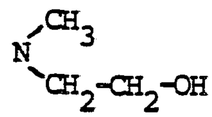 | HCl | |
| 191 | 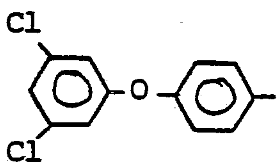 | H | H | CO | 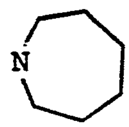 | HCl | |
| 192 | 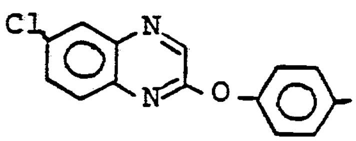 | H | H | CO | 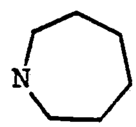 | HCl | |
| 193 | 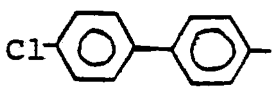 | H | H | CO | 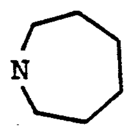 | HCl | |
| 194 | 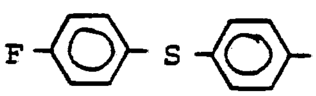 | H | H | CO | 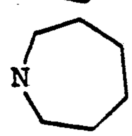 | HCl | |
| 195 | 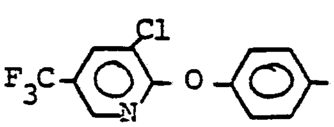 | H | H | CO | 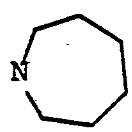 | HCl | |

Forts. Tabelle 1

| Nr. | R° | $R^1$ | $R^2$ | X | $NR^3R^4$ | HY | Fp.[°C] |
|---|---|---|---|---|---|---|---|
| 196 | 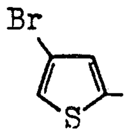 | H | H | CO | 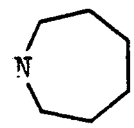 | HCl | 169-70 |
| 197 | " | H | H | CO | " | HCl | 163-64 |
| 198 | 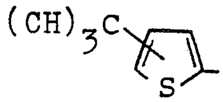 | H | H | CO | " | HCl | 148-50 |
| 199 | 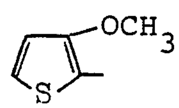 | H | H | CO | " | HCl | 153-4 |
| 200 | 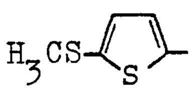 | H | H | CO | " | HCl | 135-6 |
| 201 | 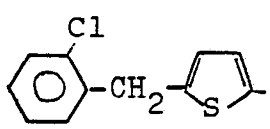 | H | H | CO | " | HCl | 147 |
| 202 | 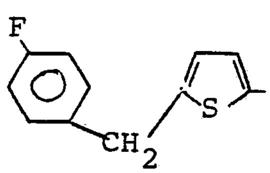 | H | H | CO | " | HCl | 156 |
| 203 | " | H | H | CO | " | HCl | 162-3 |
| 204 | 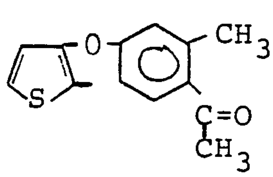 | H | H | CO | " | HCl | 175-6 |

Forts. Tabelle 1

| Nr. | R° | $R^1$ | $R^2$ | X | $NR^3R^4$ | HY | Fp.[°C] |
|---|---|---|---|---|---|---|---|
| 205 | 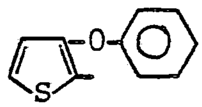 | H | H | CO | 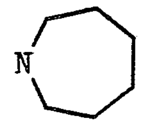 | HCl | 133-4 |
| 206 | 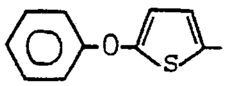 | H | H | CO | " | HCl | 140-1 |
| 207 | 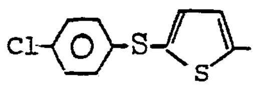 | H | H | CO | " | HCl | 154-6 |
| 208 | 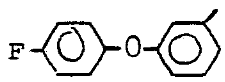 | H | H | CO | " | HCl | 158-9 |
| 209 | 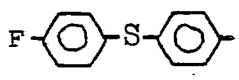 | H | H | CO | " | HCl | 152-3 |
| 210 | 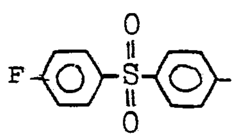 | H | H | CO | " | HCl | 157-9 |
| 211 | 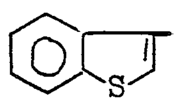 | H | H | CO | " | HCl | 195-7 |
| 212 | 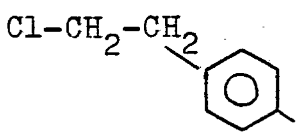 | H | H | CO | " | HCl | 147 |
| 213 | 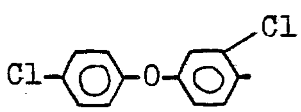 | H | H | CO | " | HCl | 134 |
| 214 | 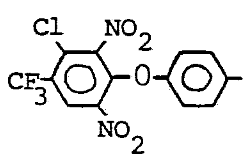 | H | H | CO | " | HCl | 180-2 |
| 215 | 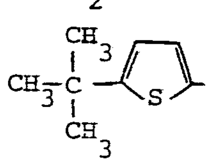 | $CH_3$ | H | CO | " | HCl | 192-3 |

Forts. Tabelle 1

| Nr. | R° | $R^1$ | $R^2$ | X | $NR^3R^4$ | HY | Fp.[°C] |
|---|---|---|---|---|---|---|---|
| 216 | 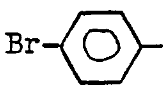 | $CH_3$ | H | CO | N | HCl | 171 |
| 217 | 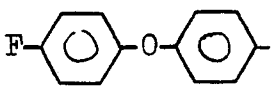 | $CH_3$ | H | CO | " | HCl | 162-4 |

*) 1 : 1 - Isomerengemisch

**) "Erythro"-Form (Diastereomerenreinheit ca. 90 %)

***) aus L-Prolinol synthetisiert

****) enthält ca. 30 % der gemäß Beispiel 111 hergestellten Verbindung

C. Biologische Beispiele .

Beispiel I:

Weinpflanzen, die aus Stecklingen der plasmoparaanfälligen Sorte Müller-Thurgau gezogen waren, wurden im 4-Blattstadium mit wäßrigen Suspensionen der beanspruchten Verbindungen tropfnaß behandelt. Die Anwendungskonzentrationen betrugen 500, 250 und 125 mg Wirkstoff pro Liter Spritzbrühe.

Nach dem Antrocknen des Spritzbelages wurden die Pflanzen mit einer Zoosporangiensuspension von Plasmopara viticola inokuliert und tropfnaß in eine Klimakammer bei einer Temperatur von 20°C und einer relativen Luftfeuchtigkeit von 100 % gestellt. Nach 24 Stunden wurden die infizierten Pflanzen der Klimakammer entnommen und in ein Gewächshaus mit einer Temperatur von 23°C und einer Luftfeuchtigkeit von ca. 80-90 % gebracht.

Nach einer Inkubationszeit von 7 Tagen wurden die Pflanzen angefeuchtet, über Nacht in die Klimakammer gestellt und die Krankheit zum Ausbruch gebracht. Anschließend erfolgte die Befallsauswertung. Der Befallsgrad wurde in % befallener Blattfläche im Vergleich zu den unbehandelten, infizierten Kontrollpflanzen ausgedrückt und ist in Tabelle II wiedergegeben.

TABELLE II

| Verbindung gem. Beispiel | % Plasmopara viticola-Befall bei mg Wirkstoff/Ltr. Spritzbrühe 500 | 250 | 125 |
|---|---|---|---|
| 1 | 0 | 0 | 0 |
| 2 | 0 | 0 | 0 |
| 3 | 0 | 0 | 0 |
| 4 | 0 | 0 | 0 |
| 10 | 0 | 0 | 0 |
| 12 | 0 | 0 | 0 |
| 16 | 0 | 0 | 0-3 |
| 17 | 0 | 0 | 0 |
| 18 | 0 | 0 | 0 |
| 20 | 0 | 0 | 0 |
| 22 | 0 | 0 | 0 |
| 23 | 0 | 0 | 0 |
| 24 | 0 | 0 | 0 |
| 27 | 0 | 0 | 0 |
| 28 | 0 | 0 | 0 |
| 29 | 0 | 0 | 0-3 |
| 31 | 0 | 0 | 0 |
| 36 | 0 | 0 | 0 |
| 37 | 0 | 0 | 0 |
| 38 | 0 | 0 | 0 |
| 39 | 0 | 0 | 0 |
| 40 | 0 | 0 | 0 |
| 41 | 0 | 0 | 0 |
| 42 | 0 | 0 | 0 |
| 43 | 0 | 0 | 0 |
| 44 | 0 | 0 | 0 |
| 45 | 0 | 0 | 0 |
| 60 | 0 | 0 | 0 |
| 66 | 0 | 0 | 0 |

Forts. Tabelle II

| Verbindung gem. Beispiel | % Plasmopara viticola-Befall bei mg Wirkstoff/Ltr. Spritzbrühe | | |
|---|---|---|---|
| | 500 | 250 | 125 |
| 69 | 0 | 0 | 0 |
| 71 | 0 | 0 | 0 |
| 77 | 0 | 0 | 0 |
| 80 | 0 | 0 | 0 |
| 84 | 0 | 0 | 0 |
| 85 | 0 | 0 | 0 - 3 |
| 89 | 0 | 0 | 0 - 3 |
| 90 | 0 | 0 | 0 |
| 91 | 0 | 0 | 0 |
| 93 | 0 | 0 | 0 |
| 94 | 0 | 0 | 0 |
| 101 | 0 | 0 | 0 |
| 102 | 0 | 0 | 0 |
| 103 | 0 | 0 | 0 |
| 105 | 0 | 0 | 0 |
| 106 | 0 | 0 | 0 |
| 107 | 0 | 0 | 0 |
| 108 | 0 | 0 | 0 |
| 109 | 0 | 0 | 0 |
| 110 | 0 | 0 | 0 |
| 111 | 0 | 0 | 0 |
| 112 | 0 | 0 | 0 |
| 113 | 0 | 0 | 0 |
| 114 | 0 | 0 | 0 |
| 117 | 0 | 0 | 0 |
| 119 | 0 | 0 | 0 |
| 123 | 0 | 0 | 0 |
| 125 | 0 | 0 | 0 |

Forts. Tabelle II

| Verbindung gem. Beispiel | % Plasmopara viticola-Befall bei mg Wirkstoff/Ltr. Spritzbrühe | | |
|---|---|---|---|
| | 500 | 250 | 125 |
| 126 | 0 | 0 | 0 |
| 127 | 0 | 0 | 0 |
| unbehandelte, infizierte Pflanzen | | 100 | |

Beispiel II

Reispflanzen wurden im 4-Blattstadium mit den beanspruchten Verbindungen in Konzentrationen von 500, 250 und 125 mg Wirkstoff pro Liter Spritzbrühe gleichmäßig behandelt. Nach dem Abtrocknen des Spritzbelages wurden die Pflanzen mit einer Sporensuspension von Piricularia oryzae gleichmäßig besprüht und für 48 h in eine dunkel gehaltene Klimakammer mit 25°C und 100 % relativer Luftfeuchte gestellt. Anschließend wurden die Pflanzen in einem Gewächshaus bei 25°C und 85 % relativer Luftfeuchte gehalten und 14 Tage nach Inokulation auf Befall mit Piricularia oryzae untersucht. Der Befallsgrad wurde ausgedrückt in % befallener Blattfläche, bezogen auf unbehandelte, infizierte Kontrollpflanzen (= 100 % Befall).

TABELLE III

| Verbindung gem. Beispiel | mit Piricularia befallene Blattfläche in % bei mg Wirkstoff/Ltr. Spritzbrühe | | |
|---|---|---|---|
| | 500 | 250 | 125 |
| 2 | 0 | 0 | 0 |
| 3 | 0 | 0 | 0 |
| 10 | 0 | 0 | 0 |
| 12 | 0 | 0 | 0 |
| 13 | 0 | 0 | 0 - 3 |
| 108 | 0 | 0 | 0 - 3 |
| 196 | 0 | 0 | 0 |
| 200 | 0 | 0 | 0 - 3 |
| 201 | 0 | 0 | 0 - 3 |
| 206 | 0 | 0 | 0 - 3 |
| unbehandelte, infizierte Pflanzen | | 100 | |

Beispiel III

Apfelunterlagen (EM IX) wurden im 4-Blattstadium mit den beanspruchten Verbindungen in den Anwendungskonzentrationen von 500, 250 und 125 mg Wirkstoff/Liter Spritzbrühe gleichmäßig behandelt.

Nach Antrocknen des Wirkstoffbelages wurden die Pflanzen mit Konidien des Apfelschorfs (Venturia inaequalis) stark infiziert und tropfnaß in eine Klimakammer gestellt, deren Temperatur 22°C und deren relative Luftfeuchtigkeit 100 % betrug. Nach einer Infektionszeit von 48 Stunden kamen die Pflanzen in ein Gewächshaus mit 18°C und einer relativen Luftfeuchtigkeit von 95 -100 %.

Nach einer Inkubationszeit von 14 Tagen wurden die Pflanzen auf Befall mit Apfelschorf (Venturia inaequalis) untersucht. Die Beurteilung des Befalls erfolgte wie üblich nach Augenschein. Der Befallsgrad der Pflanzen mit Apfelschorf wurde in % befallener Blattfläche, bezogen auf unbehandelte, infizierte Pflanzen, ausgedrückt und ist in Tabelle IV wiedergegeben.

TABELLE IV

| Verbindung gem. Beispiel | % Schorfbefall bei mg Wirkstoff/Ltr. Spritzbrühe 500 | 250 | 125 |
|---|---|---|---|
| 4 | 0 | 0 | 0 |
| 10 | 0 | 0 | 0 - 3 |
| 17 | 0 | 0 | 0 - 3 |
| 20 | 0 | 0 | 0 |
| 21 | 0 | 0 | 0 - 3 |
| 24 | 0 | 0 | 0 - 3 |
| 31 | 0 | 0 | 0 |
| 32 | 0 | 0 | 0 |
| 37 | 0 | 0 | 0 |
| 39 | 0 | 0 | 0 |
| 40 | 0 | 0 | 0 |
| 42 | 0 | 0 | 0 |
| 45 | 0 | 0 | 0 |
| 64 | 0 | 0 | 0 - 3 |
| 66 | 0 | 0 | 0 |
| 85 | 0 | 0 | 0 |
| 200 | 0 | 0 | 0 |
| 205 | 0 | 0 | 0 |
| 206 | 0 | 0 | 0 |
| 211 | 0 | 0 | 0 |

**Forts. Tabelle IV**

| Verbindung gem. Beispiel | % Schorfbefall bei mg Wirkstoff/Ltr. Spritzbrühe | | |
|---|---|---|---|
| | 500 | 250 | 125 |
| 89 | 0 | 0 | 0 |
| 91 | 0 | 0 | 0 |
| 96 | 0 | 0 | 0 - 3 |
| unbehandelte, infizierte Pfl. | | 100 | |

Beispiel IV

Zuckerrübenpflanzen wurden im 6-Blattestadium mit den beanspruchten Verbindungen in den Aufwandmengen von 500, 250 und 125 mg/l Spritzbrühe behandelt.

Nach Antrocknen des Wirkstoffbelages wurden die Pflanzen mit Konidien des Erregers der Blattfleckenkrankheit der Rübe (Cercospora beticola) stark inokuliert und tropfnaß in eine Klimakammer mit ca. 100 % relativer Luftfeuchte und 25°C gestellt. 48 Stunden später kamen die Pflanzen in ein Gewächshaus zurück. 14 Tage später wurden sie auf Befall mit der Blattfleckenkrankheit untersucht. Der Befallsgrad wurde in % befallener Blattfläche, bezogen auf unbehandelte, infizierte Kontrollpflanzen (= 100 %), ausgedrückt. Die Ergebnisse sind in Tabelle V zusammengestellt.

**TABELLE V**

| Verbindung gem. Bsp. | mit Cercospora beticola befallene Blattfläche in % bei mg Wirkst./Ltr. Spritzb. | | |
|---|---|---|---|
| | 500 | 250 | 125 |
| 3 | 0 | 0 | 0 - 3 |
| 18 | 0 | 0 | 0 |
| 22 | 0 | 0 | 0 - 3 |
| 31 | 0 | 0 | 0 - 3 |
| 32 | 0 | 0 | 0 |
| unbehandelte, infiz. Pflanzen | | 100 | |

Beispiel V

Weizenpflanzen wurden mit den beanspruchten Verbindungen in den Anwendungskonzentrationen von 500, 250 and 125 mg Wirkstoff/Liter Spritzbrühe behandelt. Nach dem Antrocknen des Wirkstoffbelages wurden die Pflanzen mit Sporen des Weizenbraunrostes (Puccinia triticina) inokuliert und tropfnaß in eine Klimakammer mit 20°C und 100 % relativer Luftfeuchte gestellt. 24 Stunden später kamen die Pflanzen in ein Gewächshaus zurück und wurden hier 14 Tage nach Inokulation auf Befall mit Weizenbraunrost untersucht. Der Befallsgrad wurde ausgedrückt in % befallener Blattfläche, bezogen auf unbehandelte, infizierte Kontrollpflanzen (= 100 % Befall). Tabelle VI zeigt die gute Wirkung der untersuchten Verbindungen.

TABELLE VI

| Verbindung gem. Beispiel | % mit Braunrost befallene Blattfläche bei mg Wirkstoff/Ltr. Spritzbrühe | | |
|---|---|---|---|
| | 500 | 250 | 125 |
| 1 | 0 | 0 | 0 |
| 2 | 0 | 0 | 0 |
| 4 | 0 | 0 | 0 - 3 |
| 18 | 0 | 0 | 0 - 3 |
| 19 | 0 | 0 | 0 |
| 20 | 0 | 0 | 0 |
| 24 | 0 | 0 | 0 |
| 26 | 0 | 0 | 0 |
| 27 | 0 | 0 | 0 |
| 28 | 0 | 0 | 0 |
| 31 | 0 | 0 | 0 |
| 37 | 0 | 0 | 0 |
| 40 | 0 | 0 | 0 |
| 41 | 0 | 0 | 0 |
| 42 | 0 | 0 | 0 |
| 43 | 0 | 0 | 0 |
| 45 | 0 | 0 | 0 |
| 60 | 0 | 0 | 0 |
| 64 | 0 | 0 | 0 |
| 66 | 0 | 0 | 0 |
| 68 | 0 | 0 | 0 |
| 69 | 0 | 0 | 0 |
| 71 | 0 | 0 | 0 - 3 |
| 80 | 0 | 0 | 0 |
| 81 | 0 | 0 | 0 - 3 |
| 82 | 0 | 0 | 0 |
| 85 | 0 | 0 | 0 - 3 |

**Forts. Tabelle VI**

| Verbindung gem. Beispiel | % mit Braunrost befallene Blattfläche bei mg Wirkstoff/Ltr. Spritzbrühe | | |
|---|---|---|---|
| | 500 | 250 | 125 |
| 87 | 0 | 0 | 0 - 3 |
| 93 | 0 | 0 | 0 |
| 94 | 0 | 0 - 3 | 0 - 3 |
| 95 | 0 | 0 | 3 |
| 103 | 0 | 0 | 0 |
| 107 | 0 | 0 | 0 |
| 109 | 0 | 0 | 0 - 3 |
| 110 | 0 | 0 | 0 |
| 112 | 0 | 0 | 0 |
| 113 | 0 | 0 | 0 |
| 114 | 0 | 0 | 0 |
| 117 | 0 | 0 | 0 |
| 119 | 0 | 0 | 0 |
| unbehandelte, infizierte Pfl. | | 100 | |

## Ansprüche

1. Fungizide Mittel, gekennzeichnet durch einen wirksamen Gehalt an einer Verbindung der Formel I,

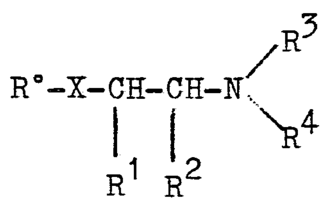

worin

R° = Phenyl, das ein-oder mehrfach substituiert ist durch Halogen, Nitro, Hydroxy, Acetoxy, $(C_1-C_{12})$-Alkyl, das gegebenenfalls ein-oder mehrfach durch Halogen, Hydroxy, $(C_1-C_4)$-Alkoxy, Phenoxy oder Phenyl substituiert ist, die beide ein-oder mehrfach durch $(C_1-C_4)$-Alkyl, Halogen, Nitro oder $CF_3$ substituiert sein können, oder

durch $(C_3-C_7)$-Cycloalkyl, das ein-oder mehrfach durch Halogen, $(C_1-C_4)$-Alkyl, $(C_1-C_4)$-Alkoxy, Phenyl oder $[(C_1-C_4)$alkoxy]carbonyl substituiert sein kann, oder

durch $(C_1-C_8)$-Alkoxy, $(C_1-C_2)$-Alkoxyethoxy, $(C_1-C_8)$-Alkylthio, $(C_1-C_8)$-Halogenalkoxy, $(C_1-C_8)$-Halogenalkylthio, $(C_2-C_4)$-Alkenyl, $(C_2-C_6)$-Alkenoxy, oder durch Phenyl, das ein- oder mehrfach durch Halogen, $CF_3$ oder $NO_2$ substituiert sein kann, oder durch Phenoxy, Benzyloxy, Naphthyloxy, Phenylthio oder Phenylsulfonyl, die alle ein-oder mehrfach durch CN, $NH_2$, $NO_2$, $COO(C_1-C_4)$alkyl, Halogen, $(C_1-C_4)$-Halogenalkyl, $(C_1-C_4)$-Alkyl, $(C_1-C_4)$Alkoxy, $(C_1-C_4)$-Alkylthio oder $(C_1-C_4)$-Halogenalkoxy, substituiert sein können,

oder durch Pyridyloxy, Chinolyloxy oder Chinoxalyloxy, die alle ein-oder mehrfach durch CN, Halogen, $(C_1$-$C_4)$-Alkyl, -$(C_1$-$C_4)$-Alkoxy, $(C_1$-$C_4)$-Halogenalkyl, $(C_1$-$C_4)$-Halogenalkoxy, Hydroxy oder Nitro substituiert sein können,

Phenyl, das an benachbarten Positionen durch $(C_1$-$C_2)$-Alkylendioxy substituiert ist,

Naphthyl, Indanyl oder 1,2,3,4-Tetrahydronaphthyl, die alle ein-oder mehrfach durch Halogen, Hydroxy, $(C_1$-$C_4)$-Alkyl, -$(C_1$-$C_4)$-Alkoxy, $(C_1$-$C_4)$-Halogenalkyl oder Phenoxy, das ein-oder mehrfach durch Halogen, $(C_1$-$C_4)$-Alkyl, $NO_2$, $(C_1$-$C_4)$-Alkoxy, $(C_1$-$C_4)$-Halogenalkyl oder $(C_1$-$C_4)$-Halogenalkoxy substituiert sein kann, substituiert sein können,

Fluorenyl oder 9,10-Dihydroanthracenyl, die beide ein-oder mehrfach durch Halogen, Hydroxy, $NO_2$, $(C_1$-$C_4)$-Alkoxy, -$(C_1$-$C_4)$-Alkyl, $(C_1$-$C_4)$-Halogenalkyl oder Phenoxy, das ein-oder mehrfach durch Halogen, $(C_1$-$C_4)$-Alkyl, CN, $NO_2$ oder $CF_3$ substituiert sein kann, substituiert sein können,

Dibenzofuranyl oder Dibenzothienyl, die beide ein-bis dreifach durch Halogen, Hydroxy, $NO_2$, $(C_1$-$C_4)$-Alkyl oder $(C_1$-$C_4)$-Alkoxy substituiert sein können,

Furanyl oder Thienyl, die beide ein-oder mehrfach durch Halogen, Nitro, $(C_1$-$C_4)$-Alkyl, $(C_1$-$C_4)$-Alkoxy, $(C_1$-$C_4)$-Hydroxyalkyl, $(C_1$-$C_4)$-Alkoxy-$(C_1$-$C_2)$-alkyl, Phenyl, Benzyl, Phenoxy oder Phenylthio, wobei diese Arylreste ein-oder mehrfach durch Halogen, $CF_3$, $NO_2$, CN, $(C_1$-$C_4)$-Alkyl oder $(C_1$-$C_4)$-Alkylcarbonyl substituiert sein können, substituiert sein können, Pyridyl, das ein-oder mehrfach durch Halogen, $NO_2$, CN, $(C_1$-$C_8)$-Alkyl, $(C_1$-$C_4)$-Halogenalkyl, $(C_1$-$C_4)$-Alkoxy, $(C_1$-$C_4)$-Halogenalkoxy, Phenoxy, das ein-oder mehrfach durch $CF_3$, $NO_2$, Halogen, $(C_1$-$C_4)$alkoxycarbonyl, $(C_1$-$C_4)$-Alkyl oder CN substituiert sein kann, Phenyl oder Phenyl, das ein-oder mehrfach durch Halogen substituiert ist, substituiert sein kann,

Benzofuranyl, Benzothienyl oder Benzofuranyl, Benzothienyl, die beide ein-bis dreifach durch Halogen, Nitro, $(C_1$-$C_4)$-Alkyl, $(C_1$-$C_4)$-Halogenalkyl oder $(C_1$-$C_4)$-Alkoxy substituiert sind,

X = $>C=O$, $>CH(OR^5)$, $>C=N-OR^5$, 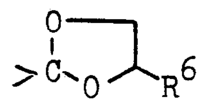

$>C=N-NH-CS-NH_2$ oder $>C=N-NH-CO-NH_2$,

$R^1$ = H, Cl, Br, $(C_1$-$C_4)$-Alkyl oder $(C_1$-$C_4)$-Alkyl, das ein-oder mehrfach durch Halogen, $(C_1$-$C_4)$-Alkoxy oder Phenyl, das ein-oder mehrfach durch Halogen substituiert sein kann, substituiert ist,

$R^2$ = H, Cl, Br, $(C_1$-$C_4)$-Alkyl, Phenyl, Phenyl, das ein-oder mehrfach durch Halogen, Nitro, Hydroxy, $(C_1$-$C_4)$-Halogenalkyl, $(C_1$-$C_4)$-Alkoxy oder Phenoxy substituiert ist, Thienyl oder Furanyl,

$R^3$ und $R^4$ unabhängig voneinander = H, $(C_1$-$C_4)$-Alkyl, $(C_1$-$C_4)$-Alkyl, das ein-oder mehrfach durch Halogen, CN, Hydroxy, $(C_1$-$C_4)$-Alkoxy, Phenoxy oder Phenyl, wobei diese Arylreste ein-oder mehrfach durch Halogen, $CF_3$, $NO_2$ oder $(C_1$-$C_4)$-Alkyl substituiert sein können, $(C_1$-$C_4$-Alkoxy)-carbonyl, Carboxy, $(C_1$-$C_4)$Alkylthio, $(C_1$-$C_4)$-Alkylsulfinyl, $(C_1$-$C_4)$-Alkylsulfonyl, $(C_3$-$C_7)$-Cycloalkyl, $(C_1$-$C_4)$-Halogenalkoxy, ( $C_1$-$C_4)$-Alkylamino oder Bis-$(C_1$-$C_4$-alkyl)amino substituiert ist,

2,2,6,6-Tetramethylpiperidin-4-yl, $(C_2$-$C_4)$-Alkenyl, $(C_2$-$C_4)$-Alkinyl oder $(C_3$-$C_7)$-Cycloalkyl, oder

$R^3$ und $R^4$ zusammen mit dem Stickstoffatom, an das sie geknüpft sind,

einen gegebenenfalls ein-oder mehrfach durch $(C_1$-$C_4)$-Alkyl, Hydroxy, $(C_1$-$C_2)$-Hydroxyalkyl, Carboxy, $(C_1$-$C_4$-alkoxy)-carbonyl, Phenyl oder Benzyl, die beide ein-oder mehrfach durch Halogen, $(C_1$-$C_4)$-Alkyl, $(C_1$-$C_4)$-Alkoxy, $NO_2$, CN, $CF_3$ oder Hydroxy substituiert sein können, oder durch $(C_1$-$C_2)$-Alkoxy-$(C_1$-$C_2)$-alkyl substituierten und/oder durch eine $(C_1$-$C_2)$-Alkylenkette einfach überbrückten 3-bis 9-gliedrigen gesättigten Heterocyclenring, der als Ringglied auch ein O-oder S-Atom oder die Gruppe $NR^7$, -CH=CH-, C=O oder -CH=N enthalten kann, .

oder 1,2,3,4-Tetrahydroisochinolin, das ein-oder mehrfach durch $(C_1$-$C_4)$-Alkyl, Hydroxy, $(C_1$-$C_2)$-Hydroxyalkyl, $(C_1$-$C_4)$-Alkoxy, $(C_1$-$C_4)$-Halogenalkyl oder Halogen substituiert sein kann, oder 8-Aza-1,4-dioxa-spiro(4,5)decan, das ein-oder mehrfach durch $(C_1$-$C_4)$-Alkyl, $(C_1$-$C_4$-Alkoxy)carbonyl, Carboxy oder $(C_1$-$C_2)$-Halogenalkyl substituiert sein kann, bedeuten,

$R^5$ = H, $(C_1$-$C_{12})$-Alkyl, $(C_2$-$C_6)$-Alkenyl, $(C_2$-$C_4)$-Alkinyl, Benzyl, das im Aromatenteil ein-oder mehrfach durch Halogen, $(C_1$-$C_4)$-Alkyl, $(C_1$-$C_2)$-Halogenalkyl, $(C_1$-$C_4)$-Alkoxy, CN, $NO_2$ oder Hydroxy substituiert sein kann, Pyridyl oder Pyridyl, das ein-oder mehrfach durch Halogen, $CF_3$, $CCl_3$ oder -$(C_1$-$C_4)$-Alkyl substituiert ist,

$R^6$ = H, $(C_1$-$C_8)$-Alkyl oder $(C_1$-$C_2)$-Halogenalkyl, und

$R^7$ = H, $(C_1$-$C_{12})$-Alkyl, $(C_1$-$C_{12})$-Alkyl, das gegebenenfalls ein-oder mehrfach durch Hydroxy oder $(C_1$-$C_2)$-Alkoxy substituiert ist, Phenyl, Benzyl oder Pyridyl, die alle ein-oder mehrfach durch Halogen, $NO_2$, OH, CN, $(C_1$-$C_4)$-Alkyl, $(C_1$-$C_2)$-Halogenalkyl oder $(C_1$-$C_4)$-Alkoxy substituiert sein können, oder den Rest -$CHR^2$-$CHR^1$-X-R°, wobei für diesen Rest die Substituenten R°, $R^1$, $R^2$ und X jeweils mit den anderen Resten R°, $R^1$, $R^2$ und X der Formel I identisch sein sollen, bedeutet, oder ihre Salze enthalten,

mit der Maßgabe, daß im Falle

a) $NR^3R^4$ = 2,6-Di-$(C_1$-$C_4$-alkyl)morpholino, X = $>C=O$ oder $>CH(OR^5)$ und R° ein substituierter Phenylring bedeutet, der einen Rest der Gruppe Halogen, $(C_1$-$C_6)$-Alkyl, $(C_5$-$C_6)$-Cycloalkyl, $(C_1$-$C_8)$-Alkoxy, $(C_2$-$C_6)$-Alkenoxy, Phenoxy oder Phenyl, wobei diese Arylreste ein-oder mehrfach durch Halogen subsituiert sein können,

enthält, oder im Falle

b) X = $>C=O$ oder $>C=N-OR^5$, $R^1$ = H oder -($C_1$-$C_4$)-Alkyl, $R^2$ = H und R° ein substituierter Phenylring bedeutet, der einen Rest der Gruppe Halogen, ($C_1$-$C_4$)-Alkyl, ($C_1$-$C_2$)-Halogenalkyl, ($C_3$-$C_7$)-Cycloalkyl, ($C_1$-$C_2$)-Alkoxy, ($C_1$-$C_2$)-Halogenalkoxy, ($C_1$-$C_2$)-Alkylthio oder ($C_1$-$C_2$)-Halogenalkylthio enthält, der Phenylring von R° des weiteren durch Hydroxy oder Nitro substituiert sein muß, und im Falle

c) $NR^3R^4$ = 2,6-Di-($C_1$-$C_4$-alkyl)morpholino und X = $>C=O$ oder $>CH(OR^5)$ bedeutet, R° keinen unsubstituierten Naphthyl-, Indanyl-oder 1,2,3,4-Tetrahydronaphthyl-Rest bedeuten darf.

2. Fungizide Mittel gemäß Anspruch 1, dadurch gekennzeichnet, daß sie eine Verbindung der Formel I von Anspruch 1, worin

R° = Phenyl, das ein-oder mehrfach substituiert ist durch Halogen, Nitro, Hydroxy, ($C_1$-$C_{12}$)-Alkyl, das gegebenenfalls ein-oder mehrfach halogeniert ist, oder Benzyl, das ein-oder mehrfach durch ($C_1$-$C_4$)-Alkyl, Halogen, Nitro oder $CF_3$ substituiert sein kann, oder durch ($C_3$-$C_7$)-Cycloalkyl, das ein-oder mehrfach durch Halogen, ($C_1$-$C_4$)-Alkyl oder Phenyl substituiert sein kann, oder

durch ($C_1$-$C_8$)-Alkoxy, ($C_1$-$C_8$)-Halogenalkoxy, oder durch Phenyl, das ein-oder mehrfach durch Halogen, $CF_3$ oder $NO_2$ substituiert sein kann, oder

durch Phenoxy, Benzyloxy, Naphthyloxy, Phenylthio, oder Phenylsulfonyl, die alle ein-oder mehrfach durch $NO_2$, Halogen, ($C_1$-$C_4$)-Halogenalkyl, ($C_1$-$C_4$)-Alkyl, ($C_1$-$C_4$)-Alkoxy, oder ($C_1$-$C_4$)-Halogenalkoxy, substituiert sein können,

oder durch Pyridyloxy, Chinolyloxy oder Chinoxalyl oxy, die alle ein-oder mehrfach durch Halogen, ($C_1$-$C_4$)-Alkyl, ($C_1$-$C_4$)-Alkoxy, ($C_1$-$C_4$)-Halogenalkyl, ($C_1$-$C_4$)-Halogenalkoxy, Hydroxy oder Nitro substituiert sein können,

Naphthyl, Indanyl oder 1,2,3,4-Tetrahydronaphthyl, die alle ein-oder zweifach durch Halogen, Hydroxy, ($C_1$-$C_4$)-Alkyl, -($C_1$-$C_4$)-Alkoxy oder ($C_1$-$C_4$)-Halogenalkyl substituiert sein können,

Fluorenyl, das ein-oder zweifach durch Halogen, $NO_2$, ($C_1$-$C_4$)-Alkyl oder ($C_1$-$C_4$)-Halogenalkyl substituiert sein kann,

Dibenzofuranyl oder Dibenzothienyl, die beide ein-bis dreifach durch Halogen, $NO_2$ oder ($C_1$-$C_4$)-Alkyl substituiert sein können,

Furanyl oder Thienyl, die beide ein-oder zweifach durch Halogen, Nitro, ($C_1$-$C_4$)-Alkyl, ($C_1$-$C_4$)-Alkoxy, substituiert sein können,

Pyridyl, das ein-oder zweifach durch Halogen, $NO_2$, CN, -($C_1$-$C_4$)-Alkyl, ($C_1$-$C_4$)-Halogenalkyl, Phenoxy, das ein-oder mehrfach durch $CF_3$, $NO_2$ oder Halogen substituiert sein kann, durch Phenyl oder Phenyl, das ein-oder zweifach durch Halogen substituiert ist, substituiert sein kann,

$R^1$, $R^2$ = H, X = CO,

$R^3$ und $R^4$ zusammen mit dem Stickstoffatom, an das sie geknüpft sind, einen gegebenenfalls ein-bis dreifach durch -($C_1$-$C_4$)-Alkyl, ($C_1$-$C_2$)-Hydroxyalkyl, Hydroxy oder ($C_1$-$C_2$)-Alkoxy-($C_1$-$C_2$)-alkyl substituierten 7-bis 9-gliedrigen Heterocyclenring, der zusätzlich die Gruppe $NR^7$ oder -(CH=N)-als Ringglied enthalten kann, und

$R^7$ = H, ($C_1$-$C_4$)-Alkyl, Phenyl oder Benzyl, die beide ein-bis dreifach durch Halogen, ($C_1$-$C_4$)-Alkyl oder ($C_1$-$C_4$)-Alkoxy substituiert sein können, bedeuten,

oder ihre Salze enthalten.

3. Verbindungen der Formel I von Anspruch 1, dadurch gekennzeichnet, daß in Formel I

R° = Phenyl, das einfach durch ($C_3$-$C_7$)-Cycloalkyl, Phenyl, Phenoxy, Naphthoxy, Benzyl, Benzyloxy, Phenylthio, Phenylsulfonyl, Pyridyloxy, Chinolyloxy oder Chinoxyalyloxy oder an benachbarten Positionen durch ($C_1$-$C_2$)-Alkylendioxy, wobei diese Reste ein-oder mehrfach durch Halogen, ($C_1$-$C_4$)-Alkyl, ($C_1$-$C_2$)-Halogenalkyl, ($C_1$-$C_4$)-Alkoxy, ($C_1$-$C_3$)-Halogenalkoxy, OH, CN, oder $NO_2$ substituiert sein können, substituiert ist und fernerein-bis dreifach durch Halogen, $NO_2$, Hydroxy, ($C_1$-$C_{12}$)-Alkyl, ($C_1$-$C_{12}$)-Alkyl, das ein-oder mehrfach durch Halogen, Hydroxy oder ($C_1$-$C_4$)-Alkoxy substituiert ist, ($C_1$-$C_8$)-Alkoxy, ($C_1$-$C_2$)-Alkoxyethoxy, ($C_1$-$C_8$)-Alkylthio, ($C_1$-$C_8$)-Halogenalkyl, ($C_1$-$C_8$)-Halogenalkoxy, ($C_1$-$C_8$)-Halogenalkylthio, ($C_2$-$C_4$)-Alkenyl, ($C_2$-$C_6$)-Alkenoxy, Phenoxy oder Phenoxy, das ein-oder mehrfach durch Halogen, $CF_3$, $NO_2$ oder ($C_1$-$C_4$)-Alkyl substituiert ist, substituiert sein kann,

Naphthyl, Indanyl oder 1,2,3,4-Tetrahydronaphthyl, wobei diese drei Reste ein-oder mehrfach durch Halogen, Hydroxy, ($C_1$-$C_4$)-Alkyl, ($C_1$-$C_4$)-Alkoxy, ($C_1$-$C_4$)-Halogenalkyl, Phenoxy oder Phenoxy, das ein-oder mehrfach durch Halogen, ($C_1$-$C_4$)-Alkyl, $NO_2$, ($C_1$-$C_4$)-Alkoxy, ($C_1$-$C_4$)-Halogenalkyl oder ($C_1$-$C_4$)-Halogenalkoxy substituiert ist, substituiert sein können,

Fluorenyl oder 9,10-Dihydroanthracenyl, die beide ein-oder mehrfach durch Halogen, Hydroxy, $NO_2$, ($C_1$-$C_4$)-Alkoxy, -($C_1$-$C_4$)-Alkyl, ($C_1$-$C_4$)-Halogenalkyl, Phenoxy oder Phenoxy, das ein-oder mehrfach durch Halogen, ($C_1$-$C_4$)-Alkyl, CN, $NO_2$ oder $CF_3$ substituiert ist, substituiert sein können,

Dibenzofuranyl oder Dibenzothienyl, die beide ein-bis dreifach durch Halogen, Hydroxy, $NO_2$, ($C_1$-$C_4$)-Alkyl oder ($C_1$-$C_4$)-Alkoxy substituiert sein können,

Furanyl oder Thienyl, die beide ein-oder mehrfach durch Halogen, Nitro, ($C_1$-$C_4$)-Alkyl, ($C_1$-$C_4$)-Hydroxyalkyl, ($C_1$-$C_4$)-Alkoxy-($C_1$-$C_2$)-alkyl, Phenyl, Benzyl, Phenoxy oder Phenylthio, wobei diese Arylreste ein-oder mehrfach durch Halogen, $CF_3$, $NO_2$, CN, ($C_1$-$C_4$)-Alkyl oder ($C_1$-$C_4$)-Alkylcarbonyl substituiert sein können, substituiert sein können,

Pyridyl oder Pyridyl, das ein-oder mehrfach durch Halogen, $NO_2$, CN, ($C_1$-$C_8$)-Alkyl, ($C_1$-$C_4$)-Halogenalkyl, ($C_1$-$C_4$)-Alkoxy, ($C_1$-$C_4$)-Halogenalkoxy, Phenoxy oder Phenoxy, das ein-oder mehrfach durch Halogen, $NO_2$, $CF_3$, COO($C_1$-$C_4$)-alkyl, CN oder ($C_1$-$C_4$)-Alkyl substituiert ist, durch Phenyl oder Phenyl, das ein-oder mehrfach durch Halogen substituiert ist, substituiert ist, oder

Benzofuranyl, Benzothienyl, die beide ein-bis dreifach, durch Halogen, Nitro, ($C_1$-$C_4$)-Alkyl, ($C_1$-$C_4$)-Halogenalkyl oder ($C_1$-$C_4$)-Alkoxy substituiert sein können,

X = $>C=O$, $>CH(OR^5)$, $>C=NOR^5$, 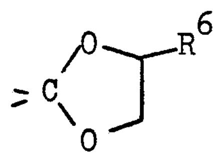 ,

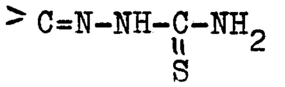 oder 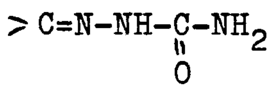 ,

$R^1$ = H, Cl, Br, ($C_1$-$C_4$)-Alkyl oder ($C_1$-$C_4$)-Alkyl, das ein-oder mehrfach durch Halogen, ($C_1$-$C_4$)-Alkoxy, Phenyl oder Phenyl, das ein-oder mehrfach durch Halogen substituiert ist, substituiert ist,

$R^2$ = H, Cl, Br, ($C_1$-$C_4$)-Alkyl, Phenyl oder Phenyl, das ein-oder mehrfach durch Halogen, Nitro, Hydroxy, ($C_1$-$C_4$)-Alkyl, ($C_1$-$C_4$)-Halogenalkyl, ($C_1$-$C_4$)-Alkoxy oder Phenoxy substituiert ist, Thienyl oder Furanyl,

$R^3$ und $R^4$ = zusammen mit dem Stickstoffatom, an das sie geknüpft sind, einen gegebenenfalls ein-oder mehrfach durch ($C_1$-$C_4$)-Alkyl, Hydroxy, ($C_1$-$C_2$)-Hydroxyalkyl oder ($C_1$-$C_2$)-Alkoxy-($C_1$-$C_2$)-alkyl substituierten und/oder durch eine ($C_1$-$C_2$)-Alkylenkette einfach überbrückten 7-bis 9-gliedrigen gesättigten Heterocyclenring, der als Ringglied auch ein Element der Gruppe O, S, $NR^7$, (CH=CH) oder (CH=N) enthalten kann, oder 8-Aza-1,4-dioxa-spiro-(4,5)decan, das ein-oder mehr-fach durch ($C_1$-$C_4$)-Alkyl, COO($C_1$-$C_4$)-alkyl COOH oder ($C_1$-$C_2$)-Halogenalkyl substituiert sein kann,

$R^5$ = H, ($C_1$-$C_{12}$)-Alkyl, ($C_2$-$C_6$)-Alkenyl, ($C_2$-$C_4$)-Alkinyl, Benzyl oder Benzyl, das im Aromatenteil ein-oder mehrfach durch Halogen, ($C_1$-$C_4$)-Alkyl, ($C_1$-$C_2$)-Halogenalkyl, ($C_1$-$C_4$)-Alkoxy, CN, $NO_2$ oder Hydroxy sub-stituiert ist, Pyridyl oder Pyridyl, das ein oder mehrfach durch Halogen, $CF_3$, $CCl_3$ oder ($C_1$-$C_4$)-Alkyl substituiert ist,

$R^6$ = H, ($C_1$-$C_8$)-Alkyl oder ($C_1$-$C_2$)-Halogenalkyl, und

$R^7$ = H, ($C_1$-$C_{12}$)-Alkyl, ($C_1$-$C_{12}$)-Alkyl, das ein-oder mehrfach durch Hydroxy oder ($C_1$-$C_2$)-Alkoxy sub stituiert ist, Phenyl, Benzyl oder Pyridyl, wobei diese Arylreste ein-oder mehrfach durch Halogen, $NO_2$, OH, CN, ($C_1$-$C_4$)-Alkyl, ($C_1$-$C_2$)-Halogenalkyl oder ($C_1$-$C_4$)-Alkoxy substituiert sein können, oder den Rest $CHR^2$-$CHR^1$-X-$R^\circ$ bedeutet, wobei die Substituenten $R^\circ$, $R^1$, $R^2$ und X in der jeweils konkreten Verbindung der Formel I die identischselben Bedeutungen besitzen sollen wie sie für die obengenannten Reste $R^\circ$, $R^1$, $R^2$ und X der neuen Verbindungen der Formel I angegeben sind.

4. Verbindungen der Formel I gemäß Anspruch 3, dadurch gekennzeichnet, daß in Formel I

$R^\circ$ = Phenyl, das einfach durch Phenyl, Phenoxy, Naphthoxy, Benzyl, Benzyloxy, Phenylthio, Phenylsulfonyl, Pyridyloxy, Chinolyloxy oder Chinoxalyloxy, die alle ein-bis dreifach durch Halogen, ($C_1$-$C_4$)-Alkyl, $CF_3$, ($C_1$-$C_4$)-Alkoxy, Hydroxy, CN oder $NO_2$ substituiert sein können, substituiert ist, und ferner ein-bis zweifach durch Halogen, Hydroxy, ($C_1$-$C_4$)-Alkyl, ($C_1$-$C_4$)-Alkoxy, oder Nitro substituiert sein kann,

Phenyl, das einfach durch ($C_3$-$C_7$)-Cycloalkyl, das ein-bis vierfach durch Halogen, Phenyl oder ($C_1$-$C_4$)-Alkyl substituiert ist, oder einfach durch ($C_1$-$C_2$)-Alkylendioxy substituiert ist, wobei Phenyl ferner ein-bis zweifach durch OH, ($C_1$-$C_4$)-Alkoxy, ($C_1$-$C_4$)-Alkyl, Phenoxy oder Phenoxy, das ein-oder mehrfach durch Halogen oder $CF_3$ substituiert ist, substituiert sein kann,

Naphthyl, Indanyl oder 1,2,3,4-Tetrahydronaphthyl, die alle ein-bis zweifach durch Halogen, Hydroxy, ($C_1$-$C_4$)-Alkyl oder ($C_1$-$C_4$)-Alkoxy substituiert sein können,

Fluorenyl, das ein-bis zweifach durch Halogen, $NO_2$ oder -($C_1$-$C_4$)-Alkyl substituiert sein kann,

Dibenzofuranyl oder Dibenzothienyl, die beide ein-bis dreifach durch $NO_2$, Halogen oder ($C_1$-$C_4$)-Alkyl substituiert sein können,

Furanyl oder Thienyl, die beide ein-bis zweifach durch Halogen, Nitro oder ($C_1$-$C_4$)-Alkyl, substituiert sein können,

Pyridyl oder Pyridyl, das ein-bis zweifach durch Halogen, $CF_3$, ($C_1$-$C_4$)-Alkyl, Phenoxy oder Phenoxy, das ein-oder mehrfach durch Halogen, $NO_2$ oder $CF_3$ substituiert ist, Phenyl oder Phenyl, das ein-bis zweifach durch Halogen substituiert ist, substituiert ist,

X = $>CO$; $R^1$, $R^2$ = H,

$R^3$ und $R^4$ = zusammen mit dem Stickstoffatom, an das sie geknüpft sind, einen gegebenenfalls ein-bis dreifach durch -($C_1$-$C_4$)-Alkyl, Hydroxy, ($C_1$-$C_2$)-Hydroxyalkyl oder ($C_1$-$C_2$)-Alkoxy-($C_1$-$C_2$)-alkyl substituierten 7-bis 9-gliedrigen Heterocyclen-Ring bedeutet, der zusätzlich die Gruppe -$NR^7$ oder -(CH=N)-als Ringglied enthalten kann, und

$R^7$ = H, ($C_1$-$C_4$)-Alkyl, Phenyl oder Benzyl, die beide ein-bis dreifach durch Halogen, ($C_1$-$C_4$)-Alkyl oder ($C_1$-$C_4$)-Alkoxy substituiert sein können, bedeuten.

5. Verfahren zur Herstellung von Verbindungen der Formel I von Ansprüchen 3 oder 4, dadurch gekennzeichnet, daß man

a) eine Verbindung der Formel II

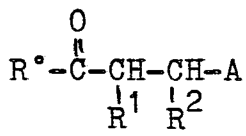 (II),

worin

A für eine nucleophil verdrängbare Abgangsgruppe steht, mit einem Amin der Formel III,

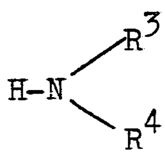 (III)

oder

b) eine Verbindung der Formel IV,

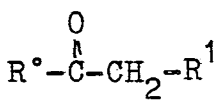 (IV)

mit einem Aldehyd der Formel V

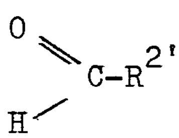 (V),

worin

$R^{2'}$ = H oder ($C_1$-$C_4$)-Alkyl bedeutet, und einem Amin der Formel III oder dessen Salz, oder

c) eine Verbindung der Formel VI,

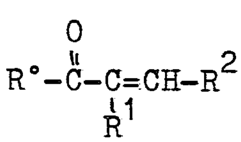 (VI)

mit einem Amin der Formel III oder dessen Salz, oder

d) eine Verbindung der Formel VII

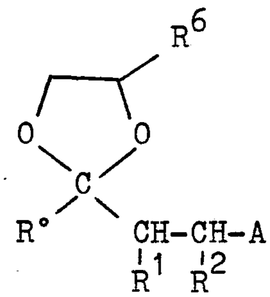

(VII)

mit einem Amin der Formel III umsetzt,

oder

die erhaltenen Verbindungen der Formel I mit X = CO, >CHOH, >C=NOH derivatisiert.

6. Verwendung von Verbindungen der Formel I gemäß Ansprüchen 1 bis 4 zur Bekämpfung von Schadpilzen.

7. Verfahren zur Bekämpfung von Schadpilzen, dadurch gekennzeichnet, daß man auf diese oder die von ihnen befallenen Flächen, Pflanzen oder Substrate eine wirksame Menge einer Verbindung der Formel I von Ansprüchen 1 bis 4 appliziert.

Patentansprüche für den Vertragsstaat : AT

1. Fungizide Mittel, gekennzeichnet durch einen wirksamen Gehalt an einer Verbindung der Formel I,

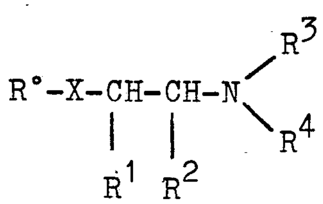

(I),

worin

R° = Phenyl, das ein-oder mehrfach substituiert ist durch Halogen, Nitro, Hydroxy, Acetoxy, $(C_1-C_{12})$-Alkyl, das gegebenenfalls ein-oder mehrfach durch Halogen, Hydroxy, $(C_1-C_4)$-Alkoxy, Phenoxy oder Phenyl substituiert ist, die beide ein-oder mehrfach durch $(C_1-C_4)$-Alkyl, Halogen, Nitro oder $CF_3$ substituiert sein können, oder

durch $(C_3-C_7)$-Cycloalkyl, das ein-oder mehrfach durch Halogen, $(C_1-C_4)$-Alkyl, $(C_1-C_4)$-Alkoxy, Phenyl oder $[(C_1-C_4)$-alkoxy]carbonyl substituiert sein kann, oder

durch $(C_1-C_8)$-Alkoxy, $(C_1-C_2)$-Alkoxyethoxy, $(C_1-C_8)$-Alkylthio, $(C_1-C_8)$-Halogenalkoxy, $(C_1-C_8)$-Halogenalkylthio, $(C_2-C_4)$-Alkenyl, $(C_2-C_6)$-Alkenoxy, oder durch Phenyl, das ein-oder mehrfach durch Halogen, $CF_3$ oder $NO_2$ substituiert sein kann, oder

durch Phenoxy, Benzyloxy, Naphthyloxy, Phenylthio oder Phenylsulfonyl, die alle ein-oder mehrfach durch CN, $NH_2$, $NO_2$, $COO(C_1-C_4)$alkyl, Halogen, $(C_1-C_4)$-Halogenalkyl, $(C_1-C_4)$-Alkyl, $(C_1-C_4)$-Alkoxy, $(C_1-C_4)$-Alkylthio oder $(C_1-C_4)$-Halogenalkoxy, substituiert sein können,

oder durch Pyridyloxy, Chinolyloxy oder Chinoxalyloxy, die alle ein-oder mehrfach durch CN, Halogen, $(C_1-C_4)$-Alkyl, -$(C_1-C_4)$-Alkoxy, $(C_1-C_4)$-Halogenalkyl, $(C_1-C_4)$-Halogenalkoxy, Hydroxy oder Nitro substituiert sein können,

Phenyl, das an benachbarten Positionen durch $(C_1-C_2)$-Alkylendioxy substituiert ist,

Naphthyl, Indanyl oder 1,2,3,4-Tetrahydronaphthyl, die alle ein-oder mehrfach durch Halogen, Hydroxy, $(C_1-C_4)$-Alkyl, -$(C_1-C_4)$-Alkoxy, $(C_1-C_4)$-Halogenalkyl oder Phenoxy, das ein-oder mehrfach durch Halogen, $(C_1-C_4)$-Alkyl, $NO_2$, $(C_1-C_4)$-Alkoxy, $(C_1-C_4)$-Halogenalkyl oder $(C_1-C_4)$-Halogenalkoxy substituiert sein kann, substituiert sein können,

Fluorenyl oder 9,10-Dihydroanthracenyl, die beide ein-oder mehrfach durch Halogen, Hydroxy, $NO_2$, $(C_1-C_4)$-Alkoxy, -$(C_1-C_4)$-Alkyl, $(C_1-C_4)$-Halogenalkyl oder Phenoxy, das ein-oder mehrfach durch Halogen, $(C_1-C_4)$-Alkyl, CN, $NO_2$ oder $CF_3$ substituiert sein kann, substituiert sein können,

Dibenzofuranyl oder Dibenzothienyl, die beide ein-bis dreifach durch Halogen, Hydroxy, $NO_2$, $(C_1-C_4)$-Alkyl oder $(C_1-C_4)$-Alkoxy substituiert sein können,

Furanyl oder Thienyl, die beide ein-oder mehrfach durch Halogen, Nitro, $(C_1-C_4)$-Alkyl, $(C_1-C_4)$-Alkoxy, $(C_1-C_4)$-Hydroxyalkyl, $(C_1-C_4)$-Alkoxy-$(C_1-C_2)$-alkyl, Phenyl, Benzyl, Phenoxy oder Phenylthio, wobei diese Arylreste ein-oder mehrfach durch Halogen, $CF_3$, $NO_2$, CN, $(C_1-C_4)$-Alkyl oder $(C_1-C_4)$-Alkylcarbonyl substituiert sein können, substituiert sein können, Pyridyl, das ein-oder mehrfach durch Halogen, $NO_2$, CN, $(C_1-C_8)$-Alkyl, $(C_1-C_4)$-Halogenalkyl, $(C_1-C_4)$-Alkoxy, $(C_1-C_4)$-Halogenalkoxy, Phenoxy, das ein-oder mehrfach durch $CF_3$, $NO_2$, Halogen, $(C_1-C_4)$-alkoxycarbonyl, $(C_1-$

$C_4$)-Alkyl oder CN substituiert sein kann,

Phenyl oder Phenyl, das ein-oder mehrfach durch Halogen substituiert ist, substituiert sein kann,

Benzofuranyl oder Benzothienyl, die beide ein-bis dreifach durch Halogen, Nitro, ($C_1$-$C_4$)Alkyl,

($C_1$-$C_4$)-Halogenalkyl oder ($C_1$-$C_4$)-Alkoxy substituiert sein können,

X = $>C{=}O$, $>CH(OR^5)$, $>C{=}N{-}OR^5$, 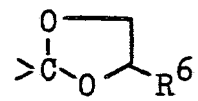

$>C{=}N{-}NH{-}CS{-}NH_2$ oder $>C{=}N{-}NH{-}CO{-}NH_2$,

$R^1$ = H, Cl, Br, ($C_1$-$C_4$)-Alkyl oder ($C_1$-$C_4$)-Alkyl, das ein-oder mehrfach durch Halogen, ($C_1$-$C_4$)-Alkoxy oder Phenyl, das ein-oder mehrfach durch Halogen substituiert sein kann, substituiert ist,

$R^2$ = H, Cl, Br, ($C_1$-$C_4$)-Alkyl, Phenyl, Phenyl, das ein-oder mehrfach durch Halogen, Nitro, Hydroxy, ($C_1$-$C_4$)-Halogenalkyl, ($C_1$-$C_4$)-Alkoxy oder Phenoxy substituiert ist, Thienyl oder Furanyl,

$R^3$ und $R^4$ unabhängig voneinander

= H, ($C_1$-$C_4$)-Alkyl, ($C_1$-$C_4$)-Alkyl, das ein-oder mehrfach durch Halogen, CN, Hydroxy, ($C_1$-$C_4$)-Alkoxy, Phenoxy oder Phenyl, wobei diese Arylreste ein-oder mehrfach durch Halogen, $CF_3$, $NO_2$ oder ($C_1$-$C_4$)-Alkyl substituiert sein können, ($C_1$-$C_4$-Alkoxy)-carbonyl, Carboxy, ($C_1$-$C_4$)-Alkylthio, ($C_1$-$C_4$)-Alkylsulfinyl, ($C_1$-$C_4$)-Alkylsulfonyl, ($C_3$-$C_7$)-Cycloalkyl, ($C_1$-$C_4$)-Halogenalkoxy, ($C_1$-$C_4$)-Alkylamino oder Bis-($C_1$-$C_4$-alkyl)amino substituiert ist,

2,2,6,6-Tetramethylpiperidin-4-yl, ($C_2$-$C_4$)-Alkenyl, ($C_2$-$C_4$)-Alkinyl oder ($C_3$-$C_7$)-Cycloalkyl, oder

$R^3$ und $R^4$ zusammen mit dem Stickstoffatom, an das sie geknüpft sind,

einen gegebenenfalls ein-oder mehrfach durch ($C_1$-$C_4$)-Alkyl, Hydroxy, ($C_1$-$C_2$)-Hydroxyalkyl, Carboxy, ($C_1$-$C_4$-alkoxy)-carbonyl, Phenyl oder Benzyl, die beide ein-oder mehrfach durch Halogen, ($C_1$-$C_4$)-Alkyl, ($C_1$-$C_4$)-Alkoxy, $NO_2$, CN, $CF_3$ oder Hydroxy substituiert sein können, oder durch ($C_1$-$C_2$)-Alkoxy-($C_1$-$C_2$)-alkyl substituierten und/oder durch eine ($C_1$-$C_2$)-Alkylenkette einfach überbrückten 3-bis 9-gliedrigen gesättigten Heterocyclenring der als Ringglied auch ein O- oder S-Atom oder die Gruppe $NR^7$, -CH=CH-, C=O oder -CH=N enthalten kann,

oder 1,2,3,4-Tetrahydroisochinolin, das ein-oder mehrfach durch ($C_1$-$C_4$)-Alkyl, Hydroxy, ($C_1$-$C_2$)-Hydroxyalkyl, ($C_1$-$C_4$)-Alkoxy, ($C_1$-$C_4$)-Halogenalkyl oder Halogen substituiert sein kann, oder 8-Aza-1,4-dioxa-spiro(4,5)decan, das ein-oder mehrfach durch ($C_1$-$C_4$)-Alkyl, ($C_1$-$C_4$-Alkoxy)carbonyl, Carboxy oder ($C_1$-$C_2$)-Halogenalkyl substituiert sein kann, bedeuten,

$R^5$ = H, ($C_1$-$C_{12}$)-Alkyl, ($C_2$-$C_6$)-Alkenyl, ($C_2$-$C_4$)-Alkinyl, Benzyl, das im Aromatenteil ein-oder mehrfach durch Halogen, ($C_1$-$C_4$)-Alkyl, ($C_1$-$C_2$)-Halogenalkyl, ($C_1$-$C_4$)-Alkoxy, CN, $NO_2$ oder Hydroxy substituiert sein kann, Pyridyl oder Pyridyl, das ein-oder mehrfach durch Halogen, $CF_3$, $CCl_3$ oder -($C_1$-$C_4$)-Alkyl substituiert ist,

$R^6$ = H, ($C_1$-$C_8$)-Alkyl oder ($C_1$-$C_2$)-Halogenalkyl, und

$R^7$ = H, ($C_1$-$C_{12}$)-Alkyl, ($C_1$-$C_{12}$)-Alkyl, das gegebenenfalls ein-oder mehrfach durch Hydroxy oder ($C_1$-$C_2$)-Alkoxy substituiert ist, Phenyl, Benzyl oder Pyridyl, die alle ein-oder mehrfach durch Halogen, $NO_2$, OH, CN, ($C_1$-$C_4$)-Alkyl, ($C_1$-$C_2$)-Halogenalkyl oder ($C_1$-$C_4$)-Alkoxy substituiert sein können, oder den Rest $-CHR^2-CHR^1-X-R°$, wobei für diesen Rest die Substituenten R°, $R^1$, $R^2$ und X jeweils mit den anderen Resten R°, $R^1$, $R^2$ und X der Formel I identisch sein sollen, bedeutet, oder ihre Salze enthalten,

mit der Maßgabe, daß im Falle

a) $NR^3R^4$ = 2,6-Di-($C_1$-$C_4$-alkyl)morpholino, X = $>C{=}O$ oder $>CH(OR^5)$ und R° ein substituierter Phenylring bedeutet, der einen Rest der Gruppe Halogen, ($C_1$-$C_6$)-Alkyl, ($C_5$-$C_6$)-Cycloalkyl, ($C_1$-$C_8$)-Alkoxy, ($C_2$-$C_6$)-Alkenoxy, Phenoxy oder Phenyl, wobei diese Arylreste ein-oder mehrfach durch Halogen substituierten sein können, enthält, oder im Falle

b) X = $>C{=}O$ oder $>C{=}N{-}OR^5$, $R^1$ = H oder ($C_1$-$C_4$)-Alkyl, $R^2$ = H und R° ein substituierter Phenylring bedeutet, der einen Rest der Gruppe Halogen, ($C_1$-$C_4$)-Alkyl, ($C_1$-$C_2$)-Halogenalkyl, ($C_3$-$C_7$)-Cycloalkyl, ($C_1$-$C_2$)-Alkoxy, ($C_1$-$C_2$)-Halogenalkoxy, ($C_1$-$C_2$)-Alkylthio oder ($C_1$-$C_2$)-Halogenalkylthio enthält, der Phenylring von R° des weiteren durch Hydroxy oder Nitro substituiert sein muß, und im Falle

c) $NR^3R^4$ = 2,6,-Di-($C_1$-$C_4$-alkyl)morpholino und X = $>C{=}O$ oder $>CH(OR^5)$ bedeutet, R° keinen unsubstituierten Naphthyl-, Indanyl-oder 1,2,3,4-Tetrahydronaphthyl-Rest bedeuten darf.

2. Fungizide Mittel gemäß Anspruch 1, dadurch gekennzeichnet, daß sie eine Verbindung der Formel 1 von Anspruch 1, worin

R° = Phenyl, das ein-oder mehrfach substituiert ist durch Halogen, Nitro, Hydroxy, ($C_1$-$C_{12}$)-Alkyl, das gegebenenfalls ein-oder mehrfach halogeniert ist, oder Benzyl, das ein-oder mehrfach durch ($C_1$-$C_4$)-Alkyl, Halogen, Nitro oder $CF_3$ substituiert sein kann, oder durch ($C_3$-$C_7$)-Cycloalkyl, das ein-

oder mehrfach durch Halogen, $(C_1\text{-}C_4)$-Alkyl, oder Phenyl substituiert sein kann, oder

durch $(C_1\text{-}C_8)$-Alkoxy, $(C_1\text{-}C_8)$-Halogenalkoxy, oder durch Phenyl, das ein-oder mehrfach durch Halogen, $CF_3$ oder $NO_2$ substituiert sein kann, oder

durch Phenoxy, Benzyloxy, Naphthyloxy, Phenylthio oder Phenylsulfonyl, die alle ein-oder mehrfach durch $NO_2$, Halogen, $(C_1\text{-}C_4)$-Halogenalkyl, $(C_1\text{-}C_4)$-Alkyl, $(C_1\text{-}C_4)$-Alkoxy, oder $(C_1\text{-}C_4)$-Halogenalkoxy, substituiert sein können,

oder durch Pyridyloxy, Chinolyloxy der Chinoxalyl oxy, die alle ein-oder mehrfach durch Halogen, $(C_1\text{-}C_4)$-Alkyl, $(C_1\text{-}C_4)$-Alkoxy, $(C_1\text{-}C_4)$-Halogenalkyl, $(C_1\text{-}C_4)$-Halogenalkoxy, Hydroxy oder Nitro substituiert sein können,

Naphthyl, Indanyl oder 1,2,3,4-Tetrahydronaphthyl, die alle ein-oder zweifach durch Halogen, Hydroxy, $(C_1\text{-}C_4)$-Alkyl, -$(C_1\text{-}C_4)$-Alkoxy oder $(C_1\text{-}C_4)$-Halogenalkyl substituiert sein können,

Fluorenyl, das ein-oder zweifach durch Halogen, $NO_2$, $(C_1\text{-}C_4)$-Alkyl oder $(C_1\text{-}C_4)$-Halogenalkyl substituiert sein kann,

Dibenzofuranyl oder Dibenzothienyl, die beide ein-bis dreifach durch Halogen, $NO_2$ oder $(C_1\text{-}C_4)$-Alkyl substituiert sein können,

Furanyl oder Thienyl, die beide ein-oder zweifach durch Halogen, Nitro, $(C_1\text{-}C_4)$-Alkyl, $(C_1\text{-}C_4)$-Alkoxy, substituiert sein können,

Pyridyl, das ein-oder zweifach durch Halogen, $NO_2$, CN, -$(C_1\text{-}C_4)$-Alkyl, $(C_1\text{-}C_4)$-Halogenalkyl, Phenoxy, das ein-oder mehrfach durch $CF_3$, $NO_2$ oder Halogen substituiert sein kann, durch Phenyl oder Phenyl, das ein-oder zweifach durch Halogen substituiert ist, substituiert sein kann,

$R^1$, $R^2$ = H, X = $>$CO,

$R^3$ und $R^4$ zusammen mit dem Stickstoffatom, an das sie geknüpft sind, einen gegebenenfalls ein-bis dreifach durch -$(C_1\text{-}C_4)$-Alkyl, $(C_1\text{-}C_2)$-Hydroxyalkyl, Hydroxy oder $(C_1\text{-}C_2)$-Alkoxy-$(C_1\text{-}C_2)$-alkyl substituierten 7-bis 9-gliedrigen Heterocyclenring, der zusätzlich die Gruppe $NR^7$ oder -(CH = N)-als Ringglied enthalten kann, und

$R^7$ = H, $(C_1\text{-}C_4)$-Alkyl, Phenyl oder Benzyl, die beide ein-bis dreifach durch Halogen, $(C_1\text{-}C_4)$-Alkyl oder $(C_1\text{-}C_4)$-Alkoxy substituiert sein können, bedeuten,

oder ihre Salze enthalten.

3. Verwendung von Verbindungen der Formel I gemäß Ansprüchen 1 oder 2 zur Bekämpfung von Schadpilzen.

4. Verfahren zur Bekämpfung von Schadpilzen, dadurch gekennzeichnet, daß man auf diese oder die von ihnen befallenen Flächen, Pflanzen oder Substrate eine wirksame Menge einer Verbindung der Formel I von Ansprüchen 1 oder 2 appliziert.